# EUROPÄISCHE PATENTSCHRIFT

(11) **EP 1 976 827 B1**
(45) Veröffentlichungstag und Bekanntmachung des Hinweises auf die Patenterteilung: **13.12.2017**
(21) Anmeldenummer: 07703642.4
(22) Anmeldetag: 04.01.2007
(51) Int. Cl.: C07C 319/14, C07C 323/58, C07C 323/59, C07C 303/24, C07C 305/06, C07D 307/32

(54) **VERFAHREN ZUR HERSTELLUNG VON METHIONIN AUS HOMOSERIN**
METHOD FOR PRODUCING METHIONINE FROM HOMOSERINE
PROCÉDÉ DE PRODUCTION DE MÉTHIONINE À PARTIR D'HOMOSÉRINE

(30) Priorität: 28.01.2006 DE 102006004063
(43) Veröffentlichungstag der Anmeldung: 08.10.2008
(73) Patentinhaber: Evonik Degussa GmbH, 45128 Essen (DE)
(72) Erfinder: HATELEY, Martin, 63741 Aschaffenburg (DE); KOBLER, Christoph, 63755 Alzenau (DE); WECKBECKER, Christoph, 63584 Gründau-Lieblos (DE); HÄUSSNER, Thomas, 63619 Bad Orb (DE); BILZ, Jürgen, 63579 Freigericht (DE); HUTHMACHER, Klaus, 63571 Gelnhausen (DE)
(86) Internationale Anmeldenummer: PCT/EP2007/050082
(87) Internationale Veröffentlichungsnummer: WO 2007/085514

(56) Entgegenhaltungen:
- WO-A-03/099777
- J.E. LIVAK, ET AL.: "Synthesis of dl-methionine" JOURNAL OF THE AMERICAN CHEMICAL SOCIETY, Bd. 67, Nr. 12, Dezember 1945 (1945-12), Seiten 2218-2220, XP002429080 AMERICAN CHEMICAL SOCIETY, WASHINGTON, DC, US in der Anmeldung erwähnt

## Beschreibung

Die vorliegende Erfindung bezieht sich auf die Herstellung von Methionin durch Kombination von biotechnologischen und chemischen Schritten.

Insbesondere bezieht sich die vorliegende Erfindung auf die fermentative Herstellung von L-Homoserin und nachfolgende chemische Umwandlung zum L-Methionin in einem oder mehreren Schritten.

Die Aminosäure Methionin wird gegenwärtig weltweit in großen Mengen industriell hergestellt und ist von beträchtlicher kommerzieller Wichtigkeit.

Methionin wird auf vielen Gebieten angewendet wie zum Beispiel für pharmazeutische, Gesundheits- und Fitnessprodukte. Insbesondere jedoch wird Methionin als Futtermitteladditiv in vielen Futtermitteln für verschiedene Nutztiere eingesetzt, wobei sowohl die racemische als auch die enantiomerenreine Form des Methionins verwendet werden können.

Im industriellen Maßstab wird Methionin chemisch über die Bucherer-Bergs-Reaktion hergestellt, die eine Variante der Strecker-Synthese darstellt. Dabei werden die Ausgangssubstanzen Methylmercaptopropionaldehyd (hergestellt aus Acrolein und Methylmercaptan), Blausäure, Ammoniak und Kohlendioxid zum 5-(2-Methyl-mercaptoethyl)-hydantoin (Methioninhydantoin) umgesetzt, dieses anschließend alkalisch zum Alkalimethioninat hydrolysiert und dann durch Neutralisation mit Säure z.B. Schwefelsäure oder Kohlensäure das Methionin freigesetzt. Verschiedene andere Methoden können auch zur Herstellung von Methionin verwendet werden wie zum Beispiel die Amidocarbonylierungsreaktion, die Hydrolyse von Proteinen oder die Fermentation.

Seitdem Methionin industriell im großen Maßstab hergestellt wird, ist es wünschenswert, einen wirtschaftlichen aber auch umweltfreundlichen Prozess zur Verfügung zu haben.

Sowohl die Strecker-Synthese als auch die Bucherer-Bergs-Reaktion haben den Nachteil, dass die giftigen Vorstufen Blausäure und Acrolein als C₁- bzw. C₃- Baustein verwendet werden. Blausäure wird aus Methan und Ammoniak bei hohen Temperaturen hergestellt. Acrolein wird durch partielle Oxidation von Propen hergestellt, das wiederum aus Erdöl gewonnen wird. Der Methionin-Prozess wird beispielsweise in EP 1256571 näher beschrieben. Der Prozess zur Herstellung von Acrolein wird beispielsweise in EP 417723 näher beschrieben. Beide Prozesse sind mit hohem technischen Aufwand und hohem Energieaufwand verbunden.

Bedingt durch den Preisanstieg beim Erdöl in den letzten Jahren ist auch Acrolein immer teurer und damit als Baustein ökonomisch immer weniger attraktiv geworden. Außerdem rufen sowohl Blausäure als auch Acrolein aufgrund ihrer Toxizität und ihren physikalischen Eigenschaften im Hinblick auf Sicherheit und Umweltschutz entsprechenden Aufwand bei der Handhabung großer Mengen hervor.

Methionin fällt bei der chemische Synthese als eine racemische Mischung aus D- und L-Enantiomerem an. Dieses Racemat kann direkt als Futtermitteladditiv eingesetzt werden, da unter in vivo-Bedingungen ein Umwandlungsmechanismus besteht, der das unnatürliche D-Enantiomer in das natürliche L-Enantiomer überführt. Mit dieser Umwandlung ist jedoch ein Verlust an Methionin verbunden, und damit auch ein Verlust an Bioeffizienz verglichen mit der gleichen Menge an reinem L-Enantiomer. Es ist also mehr racemisches D,L-Methionin erforderlich im Vergleich zu L-Methionin, um denselben Effekt zu erreichen.

Es war daher wünschenswert, einen möglichst wirtschaftlich interessanteren und umweltfreundlicheren sowie sicheren Prozess zur Herstellung von Methionin bereitzustellen. Insbesondere war wünschenswert einen Prozess zur Herstellung von enantiomerenangereichertem L-Methionin, ganz besonders bevorzugt von möglichst enantiomerenreinem L-Methionin bereitzustellen, der in industriellen Maßstab durchführbar sein sollte.

Bisherige Prozesse, die auf der Herstellung von L-Methionin mit Hilfe von Mikroorganismen beruhen, wie z.B. in der WO04/024933 beschrieben, haben den Nachteil, dass vergleichsweise geringe Ausbeuten erreicht werden. Dies hat seine Ursache insbesondere in den Problemen mit dem streng organisierten regulatorischen Netzwerk der mikrobiellen L-Methionin-Biosynthese, mit der Ausscheidung von Methionin aus der Zelle in die Fermentationsbrühe wie auch mit dem energieintensiven Achtelelektronenschritt bei der Reduktion von Sulfat zu Schwefelwasserstoff. Andererseits bewirkt die begrenzte Löslichkeit von Methionin in Wasser bzw. in wässrigen Fermentationsbrühen, dass Methionin bei hoher Biosyntheseleistung in der Fermentation ausfällt und somit die Reinigung erschwert wird. Die aufwändige Reinigung führt in der Folge dazu, dass beträchtliche Abfallströme anfallen, deren Beseitigung mit hohen Kosten verbunden sind.

In der WO05/059155 wird zwar ein Verfahren zur verbesserten Isolierung von L-Methionin aus Fermentationsbrühen beschrieben. Die Verbesserung wird jedoch durch eine vergleichsweise komplizierte Folge von Schritten, die Aufheizen und Auflösen des L-Methionins in der Fermentationsbrühe, Abfiltrieren der Biomasse bei definierter Temperatur und Nachbehandeln der abfiltrierten methioninhaltigen Biomasse, Eindampfen der Mutterlauge, Abkühlen, Kristallisieren, Abfiltrieren, Waschen und Trocknen des L-Methionins aus der Mutterlauge und Rückführung von Mutterlaugen umfassen, erkauft, und dadurch, dass zwei unterschiedliche Produktströme nämlich ein niedrig und ein hoch konzentriertes L-Methionin-Produkt anfallen. Der Zwangsanfall von zwei unterschiedlichen Methionin-Qualitäten bedeutet jedoch wiederum Mehraufwand und ist außerdem unter Marketinggesichtspunkten unerwünscht.

Die genannten Probleme führen letztlich zu einer geringeren Gesamtausbeute bei einem rein fermentativen L-Methioninverfahren verglichen mit den fermentativen Herstellungsverfahren von beispielsweise L-Lysin, welche in der Technik bereits seit vielen Jahren angewendet werden und/oder zu einem entsprechenden Mehraufwand in der fermentativen Produktion von L-Methionin.

Vor dem Hintergrund der Nachteile des Standes der Technik war es insbesondere die Aufgabe, ein Verfahren für Methionin bereitzustellen, das die oben näher bezeichneten Nachteile der Verfahren aus dem Stand der Technik überwindet. Dieses Verfahren sollte möglichst ausgehend von einer anderen verfügbaren und fermentativ herstellbaren Vorstufe auf möglichst einfache Weise und ohne Verwendung der o.g. gefährlichen Chemikalien zu L-, D- bzw. D,L-Methionin, vorzugsweise aber zu L-Methionin führen und dabei insbesondere die Nachteile der herkömmlichen chemischen Verfahren sowie der direkten biotechnologischen Herstellverfahren für Methionin überwinden.

Eine weitere Aufgabe war es, ein Herstellverfahren zur Verfügung zu stellen, das wenigstens teilweise ausgehend von natürlichen oder nachwachsenden Rohstoffen durchgeführt werden kann.

Eine dritte Aufgabe war es, ein technisch ohne weiteres durchführbares Verfahren zur Verfügung zu stellen, das L-Methionin in geeigneten Mengen und Reinheiten zugänglich macht.

**Gelöst** werden diese sowie weitere nicht explizit genannte Aufgaben, die jedoch aus den hierin diskutierten Zusammenhängen ohne weiteres ableitbar oder erschließbar sind, dadurch, dass man von einer anderen verfügbaren und fermentativ besser herstellbaren Aminosäure ausgeht, die dann über eine geeignete chemische Umwandlung ohne Verwendung der o.g. gefährlichen Chemikalien zu L-, D- bzw. D,L-Methionin, insbesondere aber zu L-Methionin umgesetzt wird. Damit werden sowohl die Nachteile der herkömmlichen chemischen Herstellungsprozesse für Methionin als auch die der herkömmlichen direkten fermentativen Herstellungsprozesse für L-Methionin überwunden. Als erfindungsgemäß geeignet hat sich die Aminosäure Homoserin erwiesen, die im Gegensatz zu Methionin über eine hohe Wasserlöslichkeit verfügt und die auch über fermentative Verfahren zugänglich ist.

Der von Livak, Britton, VanderWeele und Murray beschriebene Weg ("Synthesis of dl-methionine", Journal of the American Chemical Society,(1945), 67, 2218-20), bei dem D,L-Homoserin als Synthesezwischenprodukt vorkommt, geht zunächst aus von D,L-2-Amino-4-butyrolacton, das über D,L-Homoserin, N-Carbamoylhomoserin, 4-(2-Brommethyl)-hydantoin und 4-(2-Methylthioethyl)-hydantoin schließlich zum D,L-Methionin führt:

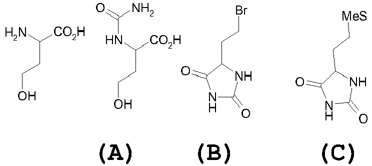

Die deuterierten Homoserinderivate HO-CHD-CH₂-CH (HNCOOtBu) COOtBu bzw. H₃CC₆H₄SO₂O-CHD-CH₂-CH(HNCOOtBu)COOtBu (tBu = tert.-Butyl)wurden gemäß Son und Woodard ("Stereochemical mechanism of iodoacetic acid mediated decomposition of L-methionine to L-homoserine lactone", Journal of the American Chemical Society (1989), 111(4), 1363-7) als Vorstufen für entsprechend in 4-Position deuteriertes L-Homoserin verwendet. Die entsprechende nichtdeuterierte Verbindungen HO-CH₂-CH₂-CH(HNCOOtBu)COOtBu bzw. H₃CC₆H₄SO₂O-CH₂-CH₂-CH(HNCOOtBu)COOtBu wurden nicht beschrieben auf dem Weg zum Homoserin.

Die nachfolgend dargestellten Verbindungen 3,6-Di(2-hydroxyethyl)-2,5-diketopiperazin, 3,6-Di(2-chlorethyl)-2,5-diketopiperazin bzw. 3,6-Di(2-methylthioethyl)-2,5-diketopiperazin stellen chemische Zwischenstufen dar, die gemäß US2,397,628 auf dem Weg zum D,L-Methionin durchlaufen wurden, allerdings nicht ausgehend vom Homoserin, sondern ausgehend von 2-Acetyl-4-butyrolacton:

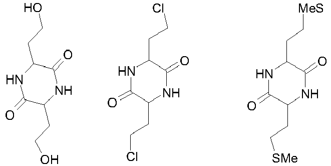

Zusätzlich gibt es weitere Herstellverfahren für D,L-Methionin, die ebenfalls nicht ausgehen von Homoserin sondern z.B. ausgehen von 2-Acetyl-4-butyrolacton über das 2-Amino-4-butyrolacton bzw. entsprechend geschütztes 2-Amino-4-butyrolacton, gemäß Snyder, Andreen, John, Cannon und Peters ("Convenient synthesis of dl-methionine", Journal of the American Chemical Society (1942), 64, 2082-4).

Die Synthese gemäß Plieninger geht aus von 2-Amino-4-butyrolacton ("Die Aufspaltung des γ-Butyrolactons und α-Amino-γ-butyrolactons mit Natriummethylmercaptid bzw. - selenid. Eine Synthese des Methionins", Chemische Berichte (1950), 83, 265-8).

Die nachfolgend dargestellten Verbindungen, 3,6-Di(2-vinyl)-2,5-diketopiperazin bzw. 3,6-Di(2-bromethyl)-2,5-diketopiperazin, stellen ebenfalls chemische Vorstufen dar, 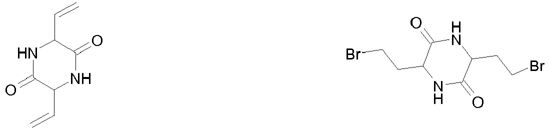 die gemäß Snyder und Chiddix ("Non-Markovnikov addition in reactions of 3,6-divinyl-2,5-diketopiperazine", Journal of the American Chemical Society (1944), 66 1002-4) auf dem Weg zum D,L-Methionin durchlaufen werden. Aber auch hier wird kein Homoserin eingesetzt.

Insbesondere gelöst werden die vorstehend genannten Aufgaben durch ein Verfahren gemäß Anspruch 1. Zweckmäßige Ausformungen und Abwandlungen des erfindungsgemäßen Verfahrens werden in den auf Anspruch 1 rückbezogenen Unteransprüchen unter Schutz gestellt.

Dadurch, dass man ein Verfahren zur Herstellung von L-Methionin, D-Methionin oder einer beliebigen Mischung von L- und D- Methionin anwendet, das von Homoserin ausgeht und bei dem das L-Homoserin, D-Homoserin oder entsprechende Mischungen von L- und D-Homoserin der nachstehenden Formel I 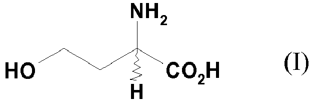 durch chemische Umwandlung in Methionin überführt werden, wobei keine der Zwischenstufen N-Carbamoylhomoserin, 4-(2-Bromethyl)-hydantoin und 4-(2-Methylthioethyl)-hydantoin (Formeln A - C) durchlaufen werden, 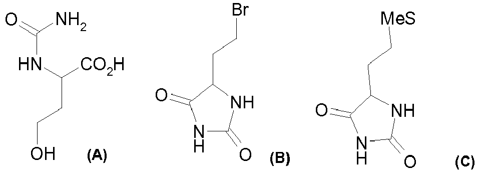 gelingt es, die Nachteile der genannten rein chemischen oder direkten biotechnologischen Verfahren zu überwinden.

Gemäß der Erfindung wird dabei das Verfahren zur chemischen Umwandlung des L- und/oder D-Homoserins zu Methionin so durchgeführt, dass in einem ersten Schritt durch sauer katalysierte Zyklisierung des entsprechenden 2-Amino-4-butyrolaceton der Formel III oder dessen Salz (Formel IV) 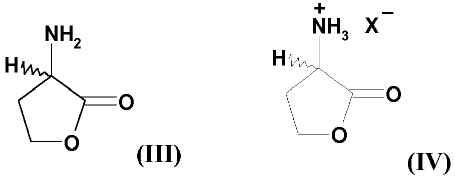 hergestellt wird, wobei X für Cl, Br, J, HSO₄, (SO₄)_{1/2}, H₂PO₄, (HPO₄)_{1/2}, (PO₄)_{1/3} oder R'-SO₃ (mit R' = Methyl, Ethyl, Phenyl, Tosyl) steht, welches dann in einem zweiten Schritt mit **MeSH** zu L-Methionin, D-Methionin oder einer entsprechenden Mischung von L- und D-Methionin umgesetzt wird.

Die oben genannten Nachteile werden insbesondere dann überwunden, wenn das eingesetzte L-Homoserin über die Fermentation hergestellt worden ist. Es ist bereits bekannt, dass L-Homoserin durch Fermentation von Mikroorganismen insbesondere Bakterien der Familie Enterobacteriaceae oder coryneforme Bakterien hergestellt werden kann, wobei Kohlenstoffquellen wie z. B. Saccharose, Glucose, Fructose und Glycerin oder Mischungen daraus und herkömmliche Stickstoffquellen wie z. B. Ammoniak verwendet werden.

Beispiele für die mikrobielle Produktion von L-Homoserin, bei denen Enterobacteriaceae, insbesondere Escherichia coli, verwendet werden, können in US 6,303,348 US 6,887,691 oder US 6,960,455 oder EP 1217076 A1 gefunden werden Beispiele für die mikrobielle Produktion von L-Homoserin, bei denen coryneforme Bakterien, insbesondere Corynebacterium glutamicum verwendet werden, können in US 3,189,526 oder US 3,598,701 gefunden werden.

Durch Einsatz von fermentativ gewonnenem L-Homoserin gelingt es, die genannten relativ gefährlichen Rohstoffe Acrolein und Blausäure zu umgehen.

Es kann aber auch vorteilhaft sein, fermentativ gewonnenes L-Homoserin mit auf klassisch chemischem Weg erzeugten racemischen D,L-Homoserin zu mischen und eine entsprechend erhaltene Mischung aus D- und L-Homoserin für die chemische Umwandlung einzusetzen, aus der am Ende dann entsprechende Gemische aus D-und L-Methionin resultieren. Dies kann v.a. dann von Vorteil sein, wenn D-/L-Homoserin als Reststoff von chemischen Herstellprozessen der D-/L-Homoserinproduktion verwertet werden soll. Auch reines D-Homoserin kann eingesetzt werden. Dies kann insbesondere dann von Vorteil sein, wenn D-Homoserin als Reststoff aus der Racematspaltung von D-/L-Homoserin verwertet werden soll. Der Einsatz von reinem D-Homoserin ist aber in der Regel nur dann vorteilhaft, wenn gezielt D-Methionin hergestellt werden soll.

Durch Einsatz von fermentativ gewonnenem L-Homoserin gelingt es hingegen direkt zum L-Methionin zu gelangen und zwar bei erfindungsgemäßer Anwendung von chemischen Verfahrensschritten, welche die L-Konfiguration nicht beeinträchtigen. Bei ausschließlichem Einsatz von L-Homoserin wird schließlich ein reines L-Methionin erzeugt, das für pharmazeutische und Lebensmittelanwendungen direkt einsetzbar ist und sich auch in der Tierernährung durch höhere Bioeffizienz im Vergleich zu herkömmlichem D,L-Methionin auszeichnet. Dieser Aspekt des erfindungsgemäßen Verfahrens ist in der Regel von größtem Nutzen.

Bei einem bevorzugten Verfahren wird ein L-Homoserinhaltiges, festes Produkt eingesetzt, das aus einer L-Homoserin-haltigen Fermentationsbrühe durch Entzug von Wasser hergestellt wurde. Dies hat den Vorteil, dass Nebenprodukte der Fermentation erst im letzten Reinigungsschritt auf der Stufe des L-Methionins abgetrennt werden können und damit Reinigungsaufwand gespart werden kann. Gegebenenfalls können Nebenprodukte und/oder Begleitstoffe der Fermentation auch im Endprodukt verbleiben, wenn sie die nachfolgende Umsetzung nicht stören oder sogar im Endprodukt erwünscht sind. Dies ist insbesondere dann der Fall, wenn sie selbst über nutritive Eigenschaften verfügen und L-Methionin für Futtermittelherstellung eingesetzt wird. Bei solchen nutritiv wirksamen Verbindungen kann es sich z.B. um weitere Aminosäuren oder Eiweißstoffe handeln.

Demgemäß ist auch ein Mischprodukt aus L-Methionin und Nebenprodukten und/oder Begleitstoffen der fermentativen Herstellung von L-Homoserin Gegenstand der Beschreibung. Die L-Homoserin enthaltende Fermentationsbrühe wird zweckmäßigerweise durch Kultivierung eines L-Homoserin ausscheidenden Mikroorganismus in einem geeigneten Nährmedium hergestellt.

Als Mikroorganismus werden vorzugsweise Bakterien, insbesondere Bakterien der Gattung Corynebacterium oder Escherichia verwendet.

Es hat sich außerdem als vorteilhaft erwiesen, wenn die Konzentration des L-Homoserins in der Fermentationsbrühe mindestens 1 g/l beträgt.

Nicht zur Erfindung gehörend wird offenbart, dass die chemische Umwandlung des L- und/oder D-Homoserins direkt mit Methylmercaptan (MeSH) ggf. in Gegenwart eines sauren Katalysators durchgeführt werden kann. Dies hat den großen Vorteil, dass ein einziger chemischer Schritt direkt zum Endprodukt L-Methionin führt. Methylmercaptan kann dabei in großen Überschüssen eingesetzt werden und unverbrauchtes Methylmercaptan anschließend leicht abgetrennt und recycliert werden, da es sich im Gegensatz zur Aminosäure um eine bei Raumtemperatur gasförmige Verbindung handelt.

Hier hat es sich als vorteilhaft erwiesen 1 bis 100, vorzugsweise 1 bis 50 Moläquivalente MeSH zu verwenden.

Um die Reaktion zu beschleunigen und die Ausbeute zu erhöhen hat es sich auch als vorteilhaft gezeigt, wenn ein saurer Katalysator ausgewählt aus der Gruppe bestehend aus Brönstedtsäuren mit einem pka von ≤ 3 verwendet wird.

Derartige Säuren sind beispielsweise HCl, HBr, HI, H₂SO₄, AlkaliHSO₄, H₃PO₄, AlkaliH₂PO₄, wobei Alkali für Lithium, Natrium, Kalium, Rubidium oder Cäsium steht, Polyphosphorsäure, C₁-C₁₂-Alkylsulfonsäure, C₆-C₁₀-Arylsulfonsäure, Trifluormethansulfonsäure, Trifluoressigsäure oder ein Copolymer aus Tetrafluorethylen und Perfluor-3,6-dioxo-4-methyl-7-octen-sulfonsäure (Nafion). Nafion als fester Katalysator hat insbesondere den Vorteil, dass es nach der Reaktion leicht aus der Reaktionsmischung abgetrennt und recycliert werden kann.

Ebenso vorteilhaft kann es sein, wenn ein **Lewissäure-Katalysator** verwendet wird. Hier sind insbesondere Lewissäure-Katalysatoren mit mindestens einer niedermolekulare Lewissäure ausgewählt aus der Gruppe AlCl₃, ZnCl₂, BF₃*OEt₂, SnCl₂, FeCl₃ zu nennen.

Auch **stark saures Ionenaustauscherharze,** die ebenfalls besonders gut zurückgewonnen werden können, haben sich hier als vorteilhaft erwiesen, insbesondere ein ggf. substituiertes z.B. mit Divinylbenzol vernetztes Polystyrolsulfonsäureharz.

Aber auch **heterogene saure Katalysatoren** aus der Gruppe Zeolith, Montmorrillonit und (WO₃-und Cs₂O)-haltiges Aluminiumoxid, und können hier verwendet werden. Bei den genannten Aluminiumoxiden werden diejenigen mit 5 -15% WO₃ und 5 -15% Cs₂O-Gehalt bevorzugt.

Zweckmäßigerweise wird die Reaktion in **Lösung und/oder in Suspension** in Gegenwart von Wasser und/oder eines organischen Lösungsmittels durchführt. Wenn man die Reaktion in Gegenwart von Wasser durchführt, kann es zweckmäßig sein, direkt von einer ggf.von Feststoffanteilen befreiten wässrigen Fermentationslösung, die L-Homoserin enthält, auszugehen, da so vorteilhafterweise weitere Aufarbeitungsschritte eingespart werden können. Aber auch ein wasserhaltiges Roh-L-Homoserin, kann entsprechend vorteilhaft eingesetzt werden.

So kann Wasser und/oder mindestens ein niedermolekulares organische Lösungsmittel ausgewählt aus der Gruppe bestehend aus C₃- bis C₆-Ketonen, vorzugsweise Methylisobutylketon (MIBK) oder Aceton, geradekettige oder verzweigte C₁- bis C₄-Alkohole, C₄- bis C₁₀-Carbonsäureester, vorzugsweise Essigsäureethylester oder -butylester, C₃- bis C₆-Carbonsäureamide, vorzugsweise DMF oder Dimethylacetamid, C₆- bis C₁₀-Aromaten, vorzugsweise Toluol und C₃- bis C₇-cyclische Carbonate, vorzugsweise Ethylencarbonat, Propylencarbonat, Butylencarbonat verwendet werden. Aber auch Methylmercaptan, in entsprechenden Überschüssen verwendet, kann als Lösungsmittel oder zumindest als Cosolvens fungieren.

Gemäß einem anderen nicht zur Erfindung gehörenden Verfahren zur chemischen Umwandlung des L- und /oder D-Homoserins zu Methionin wird in einem ersten Schritt durch Einführen einer **Abgangsgruppe Y am C₄-Atom** des Homoserins eine Verbindung der Formel II 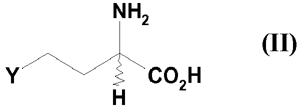 hergestellt, wobei Y steht für Halogen (= Chlor, Brom oder Jod), Sulfonyloxy (= p-Toluolsulfonyloxy [pTsO], C₆H₅SO₃, H₃CSO₃, H₅C₂SO₃ oder CF₃SO₂), Sulfat (OSO₃H) oder Phosphat (OPO₃H), und Verbindung II dann in einem zweiten Schritt mit **MeSH** zu L-Methionin, D- Methionin oder einer entsprechende Mischung von L- und D-Methionin umgesetzt.

Die Einführung der Abgangsgruppe Y geschieht vorteilhaft, wenn Y = Halogen, im ersten Schritt entsprechend durch Umsetzung des Homoserins mit PCl₅, PCl₃, BBr₃, PJ₃, POCl₃, SOCl₂ oder SOBr₂.

Wenn Y = Sulfonyloxy ist, geschieht die Einführung der Abgangsgruppe Y, im ersten Schritt entsprechend und in vorteilhafter Weise durch Umsetzung mit p-Toluolsulfonsäurechlorid (p-TsCl), C₆H₅SO₂Cl, H₃CSO₂Cl, H₅C₂SO₂Cl oder CF₃SO₂Cl.

Wenn hingegen Y = Sulphat bedeutet, werden zur Einführung der Abgangsgruppe Y, im ersten Schritt typischerweise entsprechend SO₃, H₂SO₄ oder Oleum verwendet und wenn Y = Phosphat bedeutet, wird bevorzugt Polyphoshorsäure zur Einführung von Y verwendet.

Nach der Aktivierung des Homoserins durch Einführung der entsprechende Abgangsgruppe Y in 4-Position gelingt es, in einem nächsten Schritt besonders gut die Me-S-Gruppe durch Substitution von Y einzuführen.

Diese Substitution wird vorteilhafterweise mittels Umsetzung der Verbindung der Formel II mit **MeSH** in Gegenwart eines basischen oder sauren Katalysators durchgeführt.

Als basische Katalysatoren eignen sich insbesondere NaOH, KOH, Pyridin, Trimethylamin, Triethylamin oder ein Acetat, Carbonat bzw. Hydrogencarbonat der Alkali- oder Erdalkalimetalle, wobei Alkali für Lithium, Natrium, Kalium, Rubidium oder Cäsium und Erdalkali für Magnesium, Calcium oder Barium steht.

Als saure Katalysatoren eignen sich insbesondere HCl, HBr, HI, H₂SO₄, AlkaliHSO₄, H₃PO₄, AlkaliH₂PO₄, wobei Alkali für Lithium, Natrium, Kalium, Rubidium oder Cäsium steht, Polyphosphorsäure, C₁-C₁₂-Alkylsulfonsäure, C₆-C₁₀-Arylsulfonsäure, Trifluormethansulfonsäure, Trifluoressigsäure oder ein Copolymer aus Tetrafluorethylen und Perfluor-3,6-dioxo-4-methyl-7-octen-sulfonsäure (Nafion) eingesetzt wird.

Die Umsetzung wird bevorzugt in Gegenwart eines organischen Lösungsmittels und/oder Wasser durchgeführt.

Als organisches Lösungsmittel wird dabei vorzugsweise ein niedermolekulares organische Lösungsmittel ausgewählt aus der Gruppe bestehend aus C₃- bis C₆-Ketonen, bevorzugt Methylisobutylketon (MIBK) oder Aceton, geradekettige oder verzweigte C₁- bis C₄-Alkohole, C₄- bis C₁₀-Carbonsäureester, vorzugsweise Essigsäureethylester oder -butylester, C₃- bis C₆-Carbonsäureamide, vorzugsweise DMF oder Dimethylacetamid, C₆- bis C₁₀-Aromaten, vorzugsweise Toluol und C₃- bis C₇-cyclische Carbonate, vorzugsweise Ethylencarbonat, Propylencarbonat oder Butylencarbonat verwendet.

Wie oben bereits ausgeführt wird gemäß der Erfindung das Verfahren zur chemischen Umwandlung des L- und /oder D-Homoserins zu Methionin so durchgeführt, dass in einem ersten Schritt durch **sauer katalysierte Zyklisierung** das entsprechende 2-Amino-4-butyrolacton der Formel III oder dessen Salz (Formel IV) 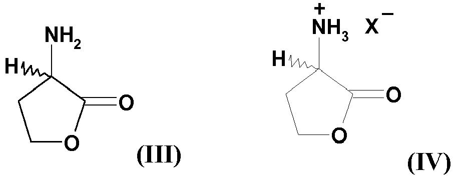 hergestellt wird, wobei X für Cl, Br, J, HSO₄, (SO₄)_{1/2}, H₂PO₄, (HPO₄)_{1/2}, (PO₄)_{1/3} oder R'-SO₃ (mit R' = Methyl, Ethyl, Phenyl, Tolyl) steht, welches dann in einem zweiten Schritt mit **MeSH** zu L-Methionin, D-Methionin oder einer entsprechenden Mischung von L- und D-Methionin umgesetzt wird. Insbesondere das Salz stellt dabei eine stabile Zwischenstufe dar, die zwischengelagert oder auch transportiert werden kann, was einen nicht unerheblichen Vorteil darstellt.

Als **saurer Katalysator** eignen sich Säuren ausgewählt aus der Gruppe bestehend aus Brönstedtsäuren mit einem pKa von < 3.

Bevorzugt eingesetzt werden als saurer Katalysator dabei HCl, HBr, HI, H₂SO₄, AlkaliHSO₄, H₃PO₄, AlkaliH₂PO₄, wobei Alkali für Lithium, Natrium, Kalium, Rubidium oder Cäsium steht, Polyphosphorsäure, C₁-C₁₂-Alkylsulfonsäure, C₆-C₁₀-Arylsulfonsäure, Trifluormethansulfonsäure, Trifluoressigsäure oder ein Copolymer aus Tetrafluorethylen und Perfluor-3,6-dioxo-4-methyl-7-octen-sulfonsäure (Nafion).

Ebenfalls eignen sich **stark saure Ionenaustauscherharze** als saurer Katalysator und hierbei insbesondere ggf. substituierte vorzugsweise mit Divinylbenzol vernetzte Polystyrolsulfonsäureharze.

Auch **heterogene saure Katalysatoren** aus der Gruppe (WO₃- und Cs₂O)-haltiges Aluminiumoxid, Zeolith und Montmorrillonit können erfindungsgemäß verwendet werden. Bei den genannten Aluminiumoxiden werden diejenigen mit 5 - 15% WO₃-Gehalt und 5 -15% Cs₂O-Gehalt bevorzugt.

Ebenso können **Lewissäure-Katalysatoren** verwendet werden und insbesondere **niedermolekulare Lewissäuren** ausgewählt aus der Gruppe AlCl₃, ZnCl₂, BF₃*OEt₂, SnCl₂, FeCl₃, welche verfügbar und kostengünstig sind.

Als nicht zur Erfindung gehörende Ausführungsform kann ein Verfahren zur chemischen Umwandlung des Homoserins zu Methionin auch so gestaltet werden, dass man die folgenden Schritte durchführt:
a) **N-Acylierung** des L- und/oder D-Homoserins mit Hilfe eines Acylierungsmittels zum N-Acyl- L- und/oder D-Homoserin der Formel V, 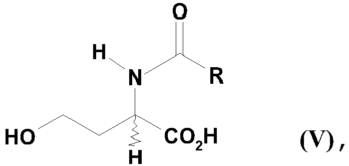 wobei R = Wasserstoff, Methyl, Ethyl, n-Propyl, i-Propyl, n-Butyl, sek.-Butyl, tert.-Butyl, Phenyl, Mono-, Di- oder Trihalogenalkyl, mit halogen = F oder Cl, vorzugsweise CF₃ oder CCl₃, Benzyloxy oder C₁- bis C₄-Alkyloxy, vorzugsweise tert.-Butyloxy oder Methyloxy, bedeutet,
b) Umsetzung des in Schritt a) erhaltenen N-Acyl-Homoserins V mit **MeSH** in Gegenwart eines basischen oder sauren Katalysators zu N-Acyl-Methionin der Formel VI 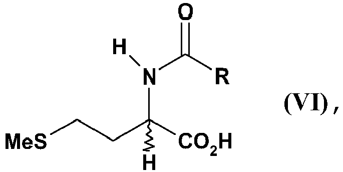
c) **Hydrolyse** des in Schritt b) erhaltenen N-Acyl-L- und/oder D-Methionins zum entsprechenden Methionin.

Je nach genauer Wahl der Umsetzungsbedingungen wird in Schritt a) entweder primär das entsprechende O-Acyl-Homoserin gebildet, das anschließend zum N-Acyl-Homoserin V umlagert, oder es wird direkt in einer Stufe V gebildet. Zur Acylierung im Schritt a) wird vorzugsweise ein Acylierungsmittel der allgemeinen Formel R-CO-X¹ verwendet, wobei X¹ = R¹COO, OR² (R² = Methyl oder Ethyl), Cl, Br sein kann und R und R¹ gleich oder verschieden sein können und Wasserstoff, Methyl, Ethyl, n-Propyl, i-Propyl, n-Butyl, sek.-Butyl, tert.-Butyl, Phenyl, Mono-, Di- oder Trihalogenalkyl, mit halogen = F oder Cl, vorzugsweise CF₃ oder CCl₃, Benzyloxy oder C₁- bis C₄-Alkyloxy, vorzugsweise tert.-Butyloxy oder Methyloxy, bedeuten. Als basischer Katalysator im Schritt b) kann NaOH, KOH, Pyridin, Trimethylamin, Triethylamin oder ein Acetat, Carbonat bzw. Hydrogencarbonat der Alkali- oder Erdalkalimetalle eingesetzt werden, wobei Alkali für Lithium, Natrium, Kalium, Rubidium oder Cäsium und Erdalkali für Magnesium, Calcium oder Barium steht.

Als saurer Katalysator für Schritt b) eignen sich insbesondere HCl, HBr, HI, H₂SO₄, AlkaliHSO₄, H₃PO₄, AlkaliH₂PO₄, wobei Alkali für Lithium, Natrium, Kalium, Rubidium oder Cäsium steht, Polyphosphorsäure, C₁-C₁₂-Alkylsulfonsäure, C₆-C₁₀-Arylsulfonsäure, Trifluormethansulfonsäure, Trifluoressigsäure oder ein Copolymer aus Tetrafluorethylen und Perfluor-3,6-dioxo-4-methyl-7-octen-sulfonsäure (Nafion).

Gemäß einer weiteren nicht zur Erfindung gehörenden Ausführungsform kann ein Verfahren zur chemischen Umwandlung des Homoserins zu Methionin auch so gestaltet werden, dass man die nachfolgenden Schritte durchführt:
a) **N-Acylierung** des L- und/oder D-Homoserins mit Hilfe eines Acylierungsmittels zum N-Acyl- L- und/oder D-Homoserin der Formel V wobei R Wasserstoff, Methyl, 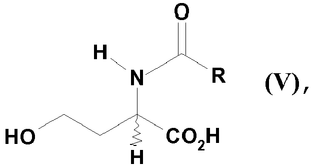 Ethyl, n-Propyl, i-Propyl, n-Butyl, sek.-Butyl, tert.-Butyl, Phenyl, Mono-, Di- oder Trihalogenalkyl, mit halogen = F oder C₁, vorzugsweise CF₃ oder CCl₃, Benzyloxy oder C₁- bis C₄-Alkyloxy, vorzugsweise tert.-Butyloxy oder Methyloxy, bedeutet,
b) Überführen der in Schritt a) erhaltenen Verbindung V durch Einführen einer **Abgangsgruppe Y am C4-Atom** in eine Verbindung der Formel VI 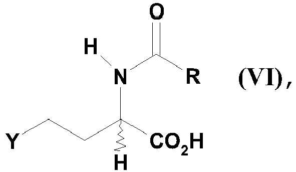 wobei Y steht für Halogen (= Chlor, Brom oder Jod), Sulfonyloxy (= pTsO, C₆H₅SO₃, H₃CSO₃ oder H_{S}C₂SO₃), Sulfat (OSO₃H) oder Phosphat (OPO₃H),
c) Umsetzung der in Schritt b) erhaltenen Verbindung VI mit MeSH in Gegenwart eines basischen oder sauren Katalysators zum N-Acyl-L-Methionin, N-Acyl-D-Methionin oder einer entsprechende Mischung von N-Acyl-L- und/oder D- Methionin der Formel VII 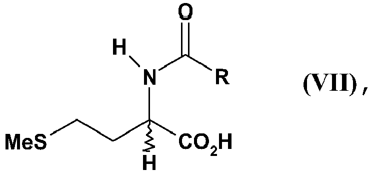
d) **Hydrolyse** des in Schritt c) erhaltenen N-Acyl-L- und/oder D-Methionins VII zu L- und/oder D-Methionin.

Die Bildung der Verbindung V geschieht dabei je nach genauer Wahl der Umsetzungsbedingungen entweder durch Umlagerung vom primär gebildetem O-Acyl-Homoserin zum N-Acyl-Homoserin oder durch eine Kombination der in situ-Lactonisierung und Acylierung mit nachfolgender Ringöffnung.

Zur Acylierung im Schritt a) wird vorzugsweise ein Acylierungsmittel der allgemeinen Formel R-CO-X¹ verwendet, wobei X¹= R¹COO, OR² (R² = Methyl oder Ethyl), Cl oder Br ist und R und R¹ gleich oder verschieden sein können und Wasserstoff, Methyl, Ethyl, n-Propyl, i-Propyl, n-Butyl, sek.-Butyl, tert.-Butyl, Phenyl, Mono-, Di- oder Trihalogenalkyl, mit halogen = F oder Cl, vorzugsweise CF₃ oder CCl₃, Benzyloxy oder C₁- bis C₄-Alkyloxy, vorzugsweise tert.-Butyloxy oder Methyloxy, bedeuten. Die Einführung der Abgangsgruppe Y geschieht vorteilhaft, wenn Y = Halogen, im ersten Schritt entsprechend durch Umsetzung des Homoserins mit PCl₃, BBr₃, PJ₃, SOCl₂ oder SOBr₂.

Wenn Y = Sulfonyloxy ist, geschieht die Einführung der Abgangsgruppe Y, im ersten Schritt entsprechend und in vorteilhafter Weise durch Umsetzung mit p-Toluolsulfonsäurechlorid (p-TsCl), C₆H₅SO₂Cl, H₃CSO₂Cl, H₅C₂SO₂Cl oder CF₃SO₂Cl. Wenn hingegen Y = Sulphat bedeutet, werden zur Einführung der Abgangsgruppe Y, im ersten Schritt typischerweise entsprechend SO₃, H₂SO₄ oder Oleum verwendet. Wenn Y = Phosphat (OPO₃H) bedeutet, wird zur Einführung der Abgangsgruppe Y, im ersten Schritt typischerweise Polyphosphorsäure verwendet.

Nach der Aktivierung des N-Acyl-Homoserins durch Einführung der entsprechende Abgangsgruppe Y in 4-Position gelingt es, in einem nächsten Schritt besonders glatt die Me-S-Gruppe durch Substitution von Y einzuführen.

Als basische Katalysatoren im Schritt c) eignen sich insbesondere NaOH, KOH, Pyridin, Trimethylamin, Triethylamin oder ein Acetat, Carbonat bzw. Hydrogencarbonat der Alkali- oder Erdalkalimetalle, wobei Alkali für Lithium, Natrium, Kalium, Rubidium oder Cäsium und Erdalkali für Magnesium, Calcium oder Barium steht. Als saure Katalysatoren im Schritt c) eignen sich insbesondere HCl, HBr, HI, H₂SO₄, AlkaliHSO₄, H₃PO₄, AlkaliH₂PO₄, wobei Alkali für Lithium, Natrium, Kalium, Rubidium oder Cäsium steht, Polyphosphorsäure, C₁-C₁₂-Alkylsulfonsäure, C₆-C₁₀-Arylsulfonsäure, Trifluormethansulfonsäure, Trifluoressigsäure oder ein Copolymer aus Tetrafluorethylen und Perfluor-3,6-dioxo-4-methyl-7-octensulfonsäure (Nafion).

Gemäß einer weiteren bevorzugten Ausführungsform der Erfindung kann ein Verfahren zur chemischen Umwandlung des L- und/oder D-Homoserins zu Methionin auch so gestaltet werden, dass man die nachfolgenden Schritte durchführt:
a) **N-Acylierung und Zyklisierung** des L- und/oder D-Homoserins mit Hilfe eines Acylierungsmittels zum N-Acyl-L- und/oder D-Homoserinlacton der Formel VIII 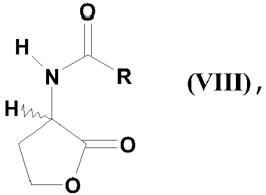 wobei R Wasserstoff, Methyl, Ethyl, n-Propyl, i-Propyl, n-Butyl, sek.-Butyl, tert.-Butyl, Phenyl, Mono-, Di- oder Trihalogenalkyl, mit halogen = F oder Cl, vorzugsweise CF₃ oder CCl₃, Benzyloxy oder C₁- bis C₄-Alkyloxy, vorzugsweise tert.-Butyloxy oder Methyloxy, bedeutet,
b) Umsetzung des in Schritt a) erhaltenen N-Acyl-Homoserinlactons mit MeSH in Gegenwart eines basischen oder sauren Katalysators zum entsprechenden N-Acyl-Methionin der Formel VII 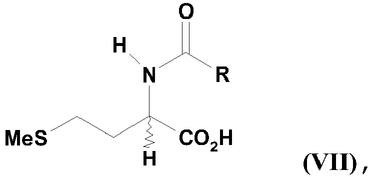
c) **Hydrolyse** des in Schritt b) erhaltenen N-Acyl- L- und/oder D-Methionins zum entsprechenden Methionin bei Temperaturen von >95 °C.

Zur Acylierung im Schritt a) wird vorzugsweise ein **Acylierungsmittel** der allgemeinen Formel R-CO-X¹ verwendet, wobei X¹= R¹COO, OR² (R² = Methyl oder Ethyl), Cl oder Br ist und R und R¹ gleich oder verschieden sein können und Wasserstoff, Methyl, Ethyl, n-Propyl, i-Propyl, n-Butyl, sek.-Butyl, tert.-Butyl, Phenyl, Mono-, Di- oder Trihalogenalkyl, mit halogen = F oder Cl , vorzugsweise CF3 oder CCl₃, Benzyloxy oder C₁- bis C₄-Alkyloxy, vorzugsweise tert.-Butyloxy oder Methyloxy, bedeuten. Die N-Acetylierung in Schritt a) geschieht dabei entweder durch Umlagerung von primär gebildetem O-Acyl-Homoserin zum N-Acyl-Homoserin mit nachfolgendem Ringschluß oder durch eine Kombination der in situ stattfindenden Lactonisierung und der direkten N-Acylierung.

Außerdem wird in der Acylierung im Schritt a) als **Lösungsmittel** bevorzugt eine Carbonsäure RCOOH oder R¹COOH verwendet, wobei R bzw. R¹ die oben angegebene Bedeutung haben, ggf. in Gegenwart eines weiteren Cosolvens aus der Gruppe bestehend aus C₃- bis C₆-Ketonen, vorzugsweise MIBK oder Aceton, C₄- bis C₁₀-Carbonsäureester, vorzugsweise Essigsäureethylester oder -butylester, C₃- bis C₆-Carbonsäureamide, vorzugsweise DMF oder Dimethylacetamid, C₆- bis C₁₀-Aromaten, vorzugsweise Toluol und C₃- bis C₇-cyclische Carbonate, vorzugsweise Ethylencarbonat Propylencarbonat oder Butylencarbonat.

Als basische Katalysatoren im Schritt a) werden bevorzugt **Pyridinderivate,** vorzugsweise Dimethylaminopyridin (DMAP), oder Carbonyldiimidazol eingesetzt.

**Schritt a) wird dabei bevorzugt bei Temperaturen** von 20 bis 100 °C, insbesondere bei 50 bis 90 °C durchgeführt.

Als **basischer Katalysator** in **Schritt b)** wird dabei bevorzugt ein Katalysator verwendet, der ausgewählt ist aus der Gruppe bestehend aus Tetraalkylammoniumhydroxiden mit max. 48 C-Atomen, Alkali- bzw. Erdalkalihydroxiden, - carbonaten, -hydrogencarbonaten, -acetaten, wobei Alkali für Lithium, Natrium, Kalium, Rubidium oder Cäsium und Erdalkali für Magnesium, Calcium oder Barium steht, tertiäre Aminen mit max. 36 C-Atomen und 1 bis 4 N-Atomen, Tetra(C₁-C₄-alkyl)-Guanidin, bicyclische Amine, vorzugsweise DBU (1,8-Diazabicyclo[5.4.0]undec-7-en) und TBD (1,5,7-Triazabicyclo[4.4.0]dec-5-en), Pyridin und stark alkalischen Ionenaustauscherharzen.

Andere bevorzugt eingesetzte **basische Katalysatoren** in **Schritt b) sind** Trialkylamine der allgemeinen Formel NR³R⁴R⁵, wobei R³, R⁴ und R⁵ gleich oder verschieden sein können und einen linearen oder verzweigten C₁- bis C₁₂-Alkylrest, vorzugsweise Methyl, Ethyl, n-Propyl, i-Propyl, n-Butyl oder sek.-Butyl bedeuten.

Ganz besonders bevorzugte basische Katalysatoren sind N(methyl)₃, N(methyl)₂(ethyl), N(methyl) (ethyl)₂, N(ethyl)₃, N(n-Propyl)3, N(ethyl) (iPropyl) oder N(n-butyl)₃, aber auch Diazabicyclooctan (DABCO), DBU, TBD, Hexamethylentetramin, Tetramethylethylendiamin oder Tetramethylguanidin.

Ebenso besonders bevorzugt werden als basische Katalysatoren verwendet R³R⁴R⁵R⁶N-hydroxid, Li-, Na-, K-, Rb-, Cs-Hydroxid, Mg-, Ca, Ba-Hydroxid, wobei R³, R⁴, R⁵ und R⁶ gleich oder verschieden sein können und einen linearen oder verzweigten C₁- bis C₁₂-Alkylrest, vorzugsweise Methyl, Ethyl, n-Propyl, i-Propyl, n-Butyl oder sek.-Butyl bedeuten.

Als besonders bevorzugte basische Katalysatoren werden auch R⁷R⁸NR⁹-substituierte, vernetzte Polystyrolharze verwendet, wobei R⁷, R⁸ und R⁹ gleich oder verschieden sein können und einen linearen oder ggf. verzweigten C₁- bis C₄-Alkylrest, vorzugsweise Methyl, Ethyl, n-Propyl, n-Butyl bedeuten.

Um eine schnellen und möglichst vollständigen Ablauf der Reaktion in Schritt b) zu erreichen, werden 1 bis 20 Moläquivalente Base, gerechnet als Hydroxid- bzw. N-Äquivalent, vorzugsweise 1 bis 10 Moläquivalente Base eingesetzt.

Wenn in **Schritt b) jedoch ein saurer Katalysator** eingesetzt wird, dann ist es vorteilhaft, einen sauren Katalysator ausgewählt aus der Gruppe bestehend aus Brönstedtsäuren mit einem pKa von < 3 oder Lewissäuren zu verwenden. Bevorzugt werden dabei als saure Katalysatoren HCl, HBr, HI, H₂SO₄, AlkaliHSO₄, H₃PO₄, AlkaliH₂PO₄ eingesetzt, wobei Alkali für Lithium, Natrium, Kalium, Rubidium oder Cäsium steht, Polyphosphorsäure, C₁-C₁₂-Alkylsulfonsäure, C₆-C₁₀-Arylsulfonsäure, Trifluormethansulfonsäure, Trifluoressigsäure oder ein Copolymer aus Tetrafluorethylen und Perfluor-3,6-dioxo-4-methyl-7-octen-sulfonsäure (Nafion).

Als saure Katalysatoren können aber auch **stark saure Ionenaustauscherharze** verwendet werden, die nach erfolgter Reaktion leicht abtrennbar sind.

Dabei bevorzugt werden ggf. substituierte, vorzugsweise mit Divinylbenzol vernetzte Polystyrolsulfon-säureharze verwendet.

Auch können **heterogene saure Katalysatoren** aus der Gruppe (WO₃- und Cs₂O)-haltiges Aluminiumoxid, Zeolith und Montmorrillonit verwendet werden. Bei den genannten Aluminiumoxiden werden diejenigen mit 5 -15% WO₃ und 5 -15% Cs₂O-Gehalt bevorzugt.

Auch **Lewissäure-Katalysatoren** finden hier in vorteilhafter Weise Verwendung.

Als Lewissäure wird dabei bevorzugt eine **niedermolekulare Lewissäure** ausgewählt aus der Gruppe AlCl₃, ZnCl₂, BF₃*OEt₂, SnCl₂, FeCl₃ verwendet.

Es ist auch vorteilhaft, wenn man die Reaktion in Schritt b) in **Lösung und/oder in Suspension** in einem organischen Lösungsmittel durchführt.

Als Lösungsmittel kann Wasser und/oder mindestens ein niedermolekulares organisches Lösungsmittel ausgewählt aus der Gruppe bestehend aus C₃- bis C₆-Ketonen, vorzugsweise MIBK oder Aceton, geradekettige oder verzweigte C₁- bis C₄-Alkohole, C₄- bis C₁₀-Carbonsäureester, vorzugsweise Essigsäure-ethylester oder -butylester, C₃- bis C₆-Carbonsäureamide, vorzugsweise DMF oder Dimethylacetamid, C₆- bis C₁₀-Aromaten, vorzugsweise Toluol und C₃- bis C₇-cyclische Carbonate, vorzugsweise Ethylencarbonat, Propylencarbonat oder Butylencarbonat verwendet werden.

Die Hydrolyse in **Schritt c)** kann in wäßriger **Lösung und/oder Suspension** durchgeführt werden.

Zusätzlich kann es jedoch auch vorteilhaft sein, wenn man zusätzlich mindestens ein niedermolekulares organisches Lösungsmittel einsetzt, das ausgewählt ist aus der Gruppe bestehend aus C₃- bis C₆-Ketonen, vorzugsweise MIBK oder Aceton, geradekettige oder verzweigte C₁- bis C₄-Alkohole, C₄- bis C₁₀-Carbonsäureester, vorzugsweise Essigsäureethylester oder -butylester, C₃- bis C₆-Carbonsäureamide, vorzugsweise DMF oder Dimethylacetamid, C₆- bis C₁₀-Aromaten, vorzugsweise Toluol und C₃- bis C₇-cyclische Carbonate, vorzugsweise Ethylencarbonat, Propylencarbonat oder Butylencarbonat.

Die Reaktion in Schritt c) wird dabei in der Regel bei einer **Temperatur** von 90 bis 180 °C, vorzugsweise bei 100 bis 160 °C, insbesondere bei 120 bis 150 °C, ganz besonders bevorzugt bei 130 bis 140 °C durchführt.

Zur Beschleunigung der Hydrolysereaktion in Schritt c) kann zusätzlich in Gegenwart eines sauren, basischen oder Lewissäure-Katalysators oder einer Kombination aus sauren und Lewissäure- Katalysator gearbeitet werden.

Ein Methioninprozess, der eine erfindungsgemäße Kombination von biotechnologischen und chemischen Schritten beinhaltet, hat insgesamt mehrere Vorteile verglichen mit einem herkömmlichen Prozess, insbesondere im Hinblick auf den erwähnten Bedarf für einen wirtschaftlicheren, sichereren Prozess, der zudem L-Methionin liefern sollte.

Erstens ermöglicht der Einsatz von Zucker anstelle von Propen (bzw. Acrolein), dass die Methioninherstellung ökonomischer gestaltet werden kann, einmal von den gegenwärtigen Rohstoffkosten her und zum anderen durch die erreichte Unabhängigkeit von den kontinuierlich steigenden Kosten für Rohöl.

Zweitens stellt der eingesetzte Zucker einen erneuerbaren Rohstoff dar, so dass hier ein wertvoller Beitrag zur Resourcenschonung geleistet wird. Außerdem sind Zucker viel weniger gefährlich als die industriellen Zwischenprodukte Acrolein und Blausäure, so dass die Substitution dieser Rohstoffe durch Zucker als Einsatzstoff das Risikopotential eines Herstellungsprozesses deutlich reduziert und damit die Sicherheit erhöht wird.

Drittens ermöglicht die Kombination eines Fermentationsschrittes, der die enantiospezifische Produktion von L-Homoserin ermöglicht, mit geeigneten vergleichsweise milden chemischen Verfahrensschritten die Umwandlung von L-Homoserin in L-Methionin ohne Racemisierung und führt auf diese Weise zu enantiomerenreinem L-Methionin. Wie erwähnt hat L-Methionin eine höhere Bioverfügbarkeit im Vergleich zu gegenwärtig produziertem D,L-Methionin.

Viertens erlaubt die Herstellung von enantiomerenreinem L-Methionin mit einem kombinierten Herstellverfahren der oben beschriebenen Art die elegante Überwindung der eingangs erwähnten Probleme, die mit der Produktion von L-Methionin auf rein biotechnologischem Weg verbunden sind.

Die nachfolgenden erfindungsgemäßen Beispiele dienen der näheren Erläuterung der Erfindung, sollen die Erfindung jedoch in keiner Weise beschränken.

### Direkte Umsetzung von L-Homoserin zu L-Methionin

### Beispiel 1 (nicht erfindungsgemäß)

Umsetzung mit einem heterogenen Katalysator (7-10%WO₃/7-10%Cs₂O auf Al₂O₃-Träger - Hersteller - Degussa.

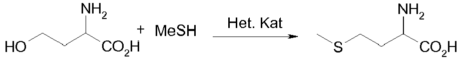

L-Homoserin (biotechnologisch hergestellt) und der fein zermahlene heterogene Katalysator wurden im Autoklav vorgelegt und MeSH wurde als Flüssigkeit dazugegeben. Der Autoklav wurde anschließend auf 140 °C über 2,5h erhitzt. Nach Entspannen und Entfernen von MeSH wurde mit einer 20 %-wässerigen NaOH Lösung gespült. Die anschließende Filtration und HPLC-Analyse ergab eine Ausbeute von 3 % d.Th. L-Methionin.

**Zum Vergleich:** Ein analoger Versuch mit reinem Al₂O₃ -Träger ergab nur Spuren von Methionin.

### Beispiel 2 - Umsetzung mit i-Propylthiol (iPrSH) und Säure/Lewissäure (fällt nicht unter die Ansprüche)

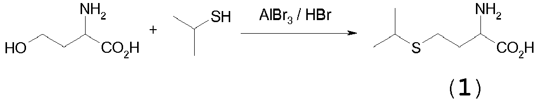

iPrSH (20 ml) wurden langsam mit HBr begast. Anschließend wurden L-Homoserin (10 mmol) zugegeben und das Gemisch 10 Minuten gerührt. Danach wurden AlCl₃ (40 mmol) zugegeben und die Reaktionsmischung 4h bei Raumtemperatur gerührt. Die Reaktionsmischung wurde mit H₂O/HCl gequencht und dann mit NaOH basisch gestellt. Nach Absaugen von Al(OH)₃ wurde die Filtratlösung zur Trockenen eingeengt und mit HPLC analysiert. Ausbeute von (1) = 8,2 %.

### Aktivierung von L-Homoserin am C-4-Atom und Umsetzung zu L-Methionin

### Beispiel 3 (nicht erfindungsgemäß)

### - Aktivierung durch Sulphat mit anschließender nukleophiler Substitution durch NaSMe:

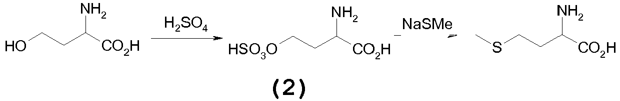

L-Homoserin (19.4 mmol) wurde mit konz. H₂SO₄ (10 ml) unter Kühlung versetzt. Das entstandene Reaktionsgemisch wurde über 30 Minuten gerührt, bis das Homoserin gelöst war. Anschließend wurde die Lösung 3 Stunden bei Raumtemperatur stehen gelassen. Danach wurde die Reaktionslösung in 800 ml auf -78 °C abgekühlten Diethylether gegeben, gut gerührt und die überstehenden Lösung abdekantiert. Der Feststoff wurde 3 mal mit je 200 ml Diethylether bei -78 °C gewaschen. Nach dem Absaugen des weiß-gelben Feststoffs wurde dieser 2 Stunden lang im Ölpumpenvakuum getrocknet. Ausbeute an Sulfatester (**2**): 88,0 %.

Der Sulfatester (19 mmol) wurde in DMSO (20 ml) gelöst und mit NaSMe (50 mmol) versetzt. Diese Reaktionslösung wurde bei 80 °C gerührt und nach 90 Minuten mittels HPLC analysiert - L-Methionin Ausbeute: 19,6 %. Wiederholung des Versuchs in N-Methylpyrrolidon (NMP) als Lösungsmittel ergab 33,6 % L-Methionin nach 10 Minuten.

### Zyklisierung von L-Homoserin und Weiterumsetzung zum L-Methionin

### Beispiel 4 - Herstellung von 2-Amino-4-butyrolacton-hydrochlorid-Salz

### Aktivierung durch Lactonbildung mit anschließender nukleophiler Substitution durch MeSH

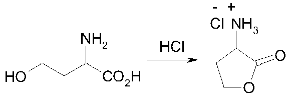

L-Homoserin (0.84 mol) wurde mit 600 ml konz. HCl (6,1 mol) versetzt. Die Lösung wurde ca.15 Minuten lang gerührt, bis sich alles aufgelöst hatte, und anschließend wurde das Wasser unter Vakuum über 1,5 Stunden entfernt. Der Rückstand wurde getrocknet. Ausbeute: 99% an 2-Amino-4-butyrolactonhydrochlorid-Salz.

### Beispiel 5 - Umsetzung des 2-Amino-4-butyrolacton-hydrochlorid-Salzes zu L-Methionin

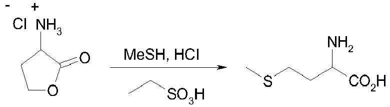

Das 2-Amino-4-butyrolactonhydrochloridsalz (22 mmol) wurde in HCl-gesättigter Ethansulfonsäure (0,2 mol) im Autoklav vorgelegt und MeSH (0,83 mol) in flüssiger Form zu dieser Mischung gegeben. Anschließend wurde der Autoklav geschlossen und 5 Stunden bei 70 °C erhitzt. Nach Entspannen und Abkühlen wurde die Reaktionslösung mittels HPLC analysiert.Die L-Methionin-Ausbeute betrug 21 %.

### Beispiel 6 - Umsetzung des 2-Amino-4-butyrolacton-hydrobromid-Salzes zu L-Methionin

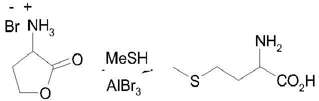

In einem Hochdruckautoklav wurde Aluminiumbromid (75 mmol) vorsichtig zu MeSH (50 ml) zugegeben. Anschließend wurde das Bromidsalz des Aminolactons (bezogen von Aldrich)(25 mmol) zugegeben. Der Autoklav wurde 1 Stunde bei Rautemperatur und danach 2 Stunden bei 40 °C geschüttelt. Der Autoklav wurde abgekühlt und entspannt. Nach Entfernen das MeSH wurde der Rückstand mit Wasser gequencht und der pH-Wert mit NaOH basisch gestellt. Der entstandenen Niederschlag wurde durch Filtration entfernt. Die Methionin-Ausbeute betrug 33 %.

### Beispiel 7 - Umsetzung des 2-Amino-4-butyrolacton-hydrochlorid-Salzes zu 2-Amino-4-methylthiobuttersäure

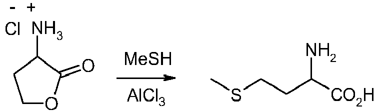

Das Chloridsalz des Aminolactons (10 mmol) sowie AlCl₃ (30 mmol) wurden in einem Autoklav vorgelegt und langsam mit MeSH (30 ml) versetzt und gerührt. Anschließend wurde die Mischung 71 Stunden bei Raumtemperatur gerührt. Nach Quenchen der Reaktionsmischung mit Wasser wurde die Ausbeute an 2-Amino-4-methylthiobuttersäure mittels HPLC als 27 % bestimmt.

### Beispiel 8 - Umsetzung des 2-Amino-4-butyrolacton-hydrochlorid-Salzes zu 2-Amino-4-isopropylthiobuttersäure (fällt nicht unter die Patentansprüche)

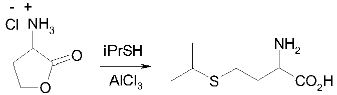

i-Propylthiol (iPrSH, 20 ml) wurde mit AlCl₃ (30 mmol) versetzt und gerührt. Anschließend wurde das Chloridsalz des Aminolactons (10 mmol) zugesetzt und die Mischung 24 Stunden bei Raumtemperatur gerührt. Nach Quenchen der Reaktionsmischung mit Wasser wurde die Ausbeute an 2-Amino-4-isopropylthiobuttersäure mittels HPLC als 77 % bestimmt.

### Beispiel 9 - Umsetzung des 2-Amino-4-butyrolactonhydrochlorid-Salzes zu L-Methionin

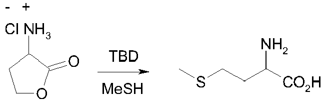

Das 2-Amino-4-butyrolacton-hydrochlorid-Salz (70 mmol) und TBD (1,5,7-Triazabicyclo[4.4.0]dec-5-en) (140 mmol) wurden im Autoklav vorgelegt und flüssiges MeSH zugegeben. Der geschlossene Autoklav wurde über 2,5 Stunden auf 70 °C erhitzt. Anschließend wurde der Autoklav, leicht gekühlt und entspannt. Das MeSH wurde entfernt und der Rückstand mittels HPLC untersucht. Die L-Methionin-Ausbeute betrug 21%.

### Beispiel 10 - Zyklisierung von L-Homoserin und N-Acylierung zum N-Acyl-2-Amino-4-butyrolacton und Weiterumsetzung zum N-Acyl-L-Methionin (Vorstufe von L-Methionin)

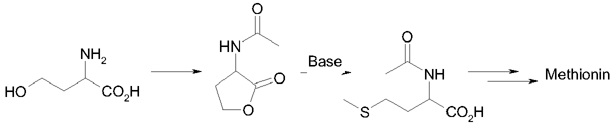

L-Homoserin (2 mol) wurde in 900 ml Acetanhydrid suspendiert und mit einer Spatelspitze Dimethylaminopyridin (DMAP) versetzt. Es wurde langsam auf 60 °C erwärmt. Nach ca. 1 Stunde stieg die Temperatur schnell auf 100 °C an. Anschließend wurde das Reaktionsgemisch 90 Minuten bei 80 °C gerührt und unter Vakuum zur Trockene eingeengt. Das erhaltenen gelbe Öl wurde in Isopropanol (600 ml) aufgenommen und übernacht bei 0 °C stehen gelassen. Die entstandenen Kristalle wurden abfiltriert, mit kaltem Isopropanol gewaschen und unter Vakuum getrocknet. Die Ausbeute betrug 60 % N-Acetyl-2-amino-4-butyrolacton isoliert, die Reinheit 99 % (gemäß HPLC).

Anschließend wurde das N-Acetyl-2-aminobutyroacton (1 eq) mit verschiedenen Basen in MeSH zu N-Acetylmethionin umgesetzt. Eine Mischung vom N-Acetylaminolacton, Base und MeSH (14 Eq) wurde in einem geschlossen Autoklav erhitzt. Nach Abkühlen, Entspannen und Entfernen von MeSH wurde das verbliebende Öl mittels HPLC untersucht. Weitere Einzelheiten sowie die erzielte Ausbeute an N-Acetyl-L-methionin sind in der Tabelle unten aufgelistet:

| Base / Fall a) bis e) | Eq bzgl. Edukt | Temperatur (°C) | Zeit (h) | Ausbeute N-Acetyl-L-met(%) |
|---|---|---|---|---|
| a) NMe₃ | 14 | 140 | 2,5 | 24,5 % |
| b) NEt₃ | 14 | 140 | 7 | 19 % |
| c) TMG* | 1 | 70 | 2,5 | 30,8 % |
| d) TMG* | 10 | 70 | 2,5 | 57,8 % |
| e) TBD** | 1 | 70 | 2,5 | 88,0 % |

| | | | | |
|---|---|---|---|---|
| * Tetramethylguanidin,** 1,5,7-Triazabicyclo[4.4.0]dec-5-en | | | | |

## Patentansprüche

1. Verfahren zur Herstellung von L-Methionin, D-Methionin oder einer beliebigen Mischung von L- und D- Methionin ausgehend von Homoserin **dadurch gekennzeichnet,**
**dass** L-Homoserin, D-Homoserin oder entsprechende Mischungen von L- und D-Homoserin der Formel I 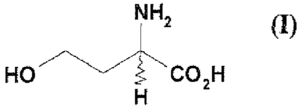 durch chemische Umwandlung in Methionin überführt werden, wobei keine der Zwischenstufen N-Carbamoylhomoserin, 4-(2-Bromethyl)-hydantoin und 4-(2-Methylthioethyl)-hydantoin durchlaufen werden und dass die chemische Umwandlung des L- und /oder D-Homoserins so durchgeführt wird, dass in einem ersten Schritt durch **sauer katalysierte Zyklisierung** das entsprechende 2-Amino-4-butyrolacton der Formel III oder dessen Salz (Formel IV) 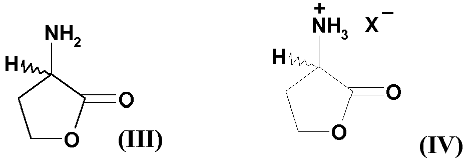 hergestellt wird, wobei X für Cl, Br, J, HSO₄, (SO₄)_{1/2}, H₂PO₄, (HPO₄)_{1/2,} (PO₄)_{1/3} oder R'-SO₃ (mit R' = Methyl, Ethyl, Phenyl, Tosyl) steht, welches dann in einem zweiten Schritt mit **MeSH** zu L-Methionin, D-Methionin oder einer entsprechenden Mischung von L- und D-Methionin umgesetzt wird.

2. Verfahren gemäß Anspruch 1, **dadurch gekennzeichnet, dass** ein **saurer Katalysator** ausgewählt aus der Gruppe bestehend aus Brönstedtsäuren mit einem pKa von ≤ 3 verwendet wird.

3. Verfahren gemäß Anspruch 2, **dadurch gekennzeichnet, dass** als saurer Katalysator HCl, HBr, HI, H₂SO₄, AlkaliHSO₄, H₃PO₄, AlkaliH₂PO₄, wobei Alkali für Lithium, Natrium, Kalium, Rubidium oder Cäsium steht, Polyphosphorsäure, C₁-C₁₂-Alkylsulfonsäure, C₆-C₁₀-Arylsulfonsäure, Trifluormethansulfonsäure, Trifluoressigsäure oder ein Copolymer aus Tetrafluorethylen und Perfluor-3,6-dioxo-4-methyl-7-octen-sulfonsäure (Nafion) eingesetzt wird.

4. Verfahren gemäß Anspruch 1, **dadurch gekennzeichnet, dass** als saurer Katalysator ein **stark saures Ionenaustauscherharz** verwendet wird.

5. Verfahren gemäß Anspruch 4, **dadurch gekennzeichnet, dass** als saurer Katalysator ein ggf. substituiertes vorzugsweise mit Divinylbenzol vernetztes Polystyrolsulfonsäureharz verwendet wird.

6. Verfahren gemäß Anspruch 1, **dadurch gekennzeichnet, dass** ein **heterogener saurer Katalysator** aus der Gruppe (WO₃- und Cs₂O)-haltiges Aluminiumoxid, Zeolith und Montmorrillonit verwendet wird.

7. Verfahren gemäß Anspruch 1, **dadurch gekennzeichnet, dass** ein **Lewissäure-Katalysator** verwendet wird.

8. Verfahren gemäß Anspruch 7, **dadurch gekennzeichnet, dass** als Lewissäure Katalysator mindestens eine **niedermolekulare Lewissäure** ausgewählt aus der Gruppe AlCl₃, ZnCl₂, BF₃*OEt₂, SnCl₂, FeCl₃ verwendet wird.

9. Verfahren zur Herstellung von L-Methionin, D-Methionin oder einer beliebigen Mischung von L- und D- Methionin ausgehend von Homoserin **dadurch gekennzeichnet, dass** L-Homoserin, D-Homoserin oder entsprechende Mischungen von L- und D-Homoserin der Formel I 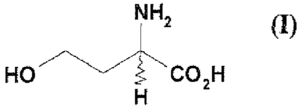 durch chemische Umwandlung in Methionin überführt werden, wobei keine der Zwischenstufen N-Carbamoylhomoserin, 4-(2-Bromethyl)-hydantoin und 4-(2-Methylthioethyl)-hydantoin durchlaufen werden und dass die chemische Umwandlung des L- und/oder D-Homoserins so durchgeführt wird, dass man die folgenden Schritte durchführt:
a) **N-Acylierung und Zyklisierung** mit Hilfe eines Acylierungsmittels zum N-Acyl-L- und/oder D-Homoserinlacton der Formel VIII 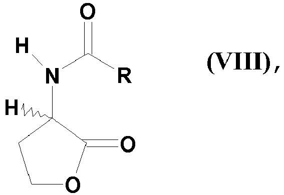 wobei R Wasserstoff, Methyl, Ethyl, n-Propyl, i-Propyl, n-Butyl, sek.-Butyl, tert.-Butyl, Phenyl, Mono-, Di- oder Trihalogenalkyl, mit halogen = F oder Cl, vorzugsweise CF₃ oder CCl₃, Benzyloxy oder C₁- bis C₄-Alkyloxy, vorzugsweise tert.-Butyloxy oder Methyloxy, bedeutet,
b) Umsetzung des in Schritt a) erhaltenen N-Acyl-Homoserinlactons mit MeSH in Gegenwart eines basischen oder sauren Katalysators zu N-Acyl-Methionin der Formel VII 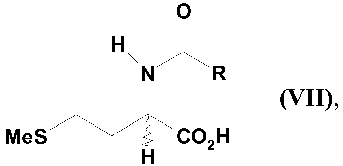
c) **Hydrolyse** des in Schritt b) erhaltenen N-Acyl-Methionins zu Methionin bei Temperaturen von >95 °C.

10. Verfahren gemäß Anspruch 9, **dadurch gekennzeichnet, dass** im Schritt a) ein **Acylierungsmittel** der allgemeinen Formel R-CO-X¹ verwendet wird, wobei X¹ = R¹COO, OR² (R² = Methyl oder Ethyl), Cl oder Br ist und R und R¹ gleich oder verschieden sein können und Wasserstoff, Methyl, Ethyl, n-Propyl, i-Propyl, n-Butyl, sek.-Butyl, tert.-Butyl, Phenyl, Mono-, Di- oder Trihalogenalkyl, mit halogen = F oder Cl, vorzugsweise CF₃ oder CCl₃, Benzyloxy oder C₁- bis C₄-Alkyloxy, vorzugsweise tert.-Butyloxy oder Methyloxy, bedeuten.

11. Verfahren gemäß Anspruch 9 oder 10, **dadurch gekennzeichnet, dass** in Schritt a) als **Lösungsmittel** eine Carbonsäure RCOOH oder R¹COOH, wobei R bzw. R¹ die oben angegebene Bedeutung haben, ggf. in Gegenwart eines weiteren Cosolvens aus der Gruppe bestehend aus C₃- bis C₆-Ketonen, vorzugsweise MIBK oder Aceton, C₄-bis C₁₀-Carbonsäureester, vorzugsweise Essigsäureethylester oder -butylester, C₃- bis C₆-Carbonsäureamide, vorzugsweise DMF oder Dimethylacetamid, C₆- bis C₁₀-Aromaten, vorzugsweise Toluol und C₃- bis C₇-cyclische Carbonate, vorzugsweise Ethylencarbonat Propylencarbonat oder Butylencarbonat, verwendet wird.

12. Verfahren gemäß einem oder mehreren der Ansprüche 9 bis 11, **dadurch gekennzeichnet, dass** in Schritt a) ein **Pyridinderivat,** vorzugsweise Dimethylaminopyridin (DMAP) oder Carbonyldiimidazol, als Katalysator eingesetzt wird.

13. Verfahren gemäß einem oder mehreren der Ansprüche 9 bis 12, **dadurch gekennzeichnet, dass Schritt a) bei Temperaturen** von 20 bis 100 °C, vorzugsweise bei 50 bis 90 °C durchgeführt wird.

14. Verfahren gemäß einem oder mehreren der Ansprüche 9 bis 13, **dadurch gekennzeichnet, dass** in **Schritt b) ein basischer Katalysator** ausgewählt aus der Gruppe bestehend aus Tetraalkylammoniumhydroxiden mit max. 48 C-Atomen, Alkali- bzw. Erdalkalihydroxiden, - carbonaten, -hydrogencarbonaten, -acetaten, wobei Alkali für Lithium, Natrium, Kalium, Rubidium oder Cäsium und Erdalkali für Magnesium, Calcium oder Barium steht, tertiäre Aminen mit max. 36 C-Atomen und 1 bis 4 N-Atomen, Tetra(C₁-C₄-alkyl)-Guanidin, bicyclische Amine, vorzugsweise DBU (1,8-Diazabicyclo[5.4.0]undec-7-en) und TBD (1,5,7-Triazabicyclo[4.4.0]dec-5-en), und stark alkalischen Ionenaustauscherharzen sowie Pyridin verwendet wird.

15. Verfahren gemäß Anspruch 14, **dadurch gekennzeichnet, dass als basischer Katalysator** ein Trialkylamin der allgemeinen Formel NR³R⁴R⁵ verwendet wird, wobei R³, R⁴ und R⁵ gleich oder verschieden sein können und einen linearen oder verzweigten C₁- bis C₁₂-Alkylrest, vorzugsweise Methyl, Ethyl, n-Propyl, i-Propyl, n-Butyl, oder sek.-Butyl bedeuten.

16. Verfahren gemäß Anspruch 15, **dadurch gekennzeichnet, dass** als basischer Katalysator N(methyl)₃, N(methyl)₂(ethyl), N(methyl) (ethyl)₂, N(ethyl)₃, N(n-Propyl)₃, N(ethyl)(iPropyl)₂ oder N(n-butyl)₃ verwendet wird.

17. Verfahren gemäß Anspruch 14, **dadurch gekennzeichnet, dass** als basischer Katalysator DABCO, DBU, TBD, Hexamethylentetramin, Tetramethylethylenediamin oder Tetramethylguanidin verwendet wird.

18. Verfahren gemäß Anspruch 14, **dadurch gekennzeichnet, dass** als basischer Katalysator R³R⁴R⁵R⁶N-hydroxid, Li-, Na-, K-, Rb-, Cs-Hydroxid, Mg-, Ca, Ba-Hydroxid verwendet wird, wobei R³, R⁴, R⁵ und R⁶ gleich oder verschieden sein können und einen linearen oder verzweigten C₁ bis C₁₂-Alkylrest, vorzugsweise Methyl, Ethyl, n-Propyl, i-Propyl, n-Butyl oder sek.-Butyl bedeuten.

19. Verfahren gemäß Anspruch 14, **dadurch gekennzeichnet, dass** als alkalischer Ionenaustauscher ein R⁷R⁸NR⁹-substituiertes vernetztes Polystyrolharz verwendet wird, wobei R⁷, R⁸ und R⁹ gleich oder verschieden sein können und einen linearen oder ggf. verzweigten C₁-bis C₄-Alkylrest, vorzugsweise Methyl, Ethyl, n-Propyl, n-Butyl bedeuten.

20. Verfahren gemäß einem oder mehreren der Ansprüche 9 bis 19, **dadurch gekennzeichnet,**
**dass** in Schritt b)1 bis 20 Moläquivalente Base, gerechnet als Hydroxid- bzw. N-Äquivalent, vorzugsweise 1 bis 10 eingesetzt werden.

21. Verfahren gemäß einem oder mehreren der Ansprüche 9 bis 19, **dadurch gekennzeichnet, dass** in **Schritt b) ein saurer Katalysator** ausgewählt aus der Gruppe bestehend aus Brönstedtsäuren mit einem pKa von < 3 oder Lewissäuren verwendet wird.

22. Verfahren gemäß Anspruch 21, **dadurch gekennzeichnet, dass** als saurer Katalysator HCl, HBr, HI, H₂SO₄, AlkaliHSO₄, H₃PO₄, AlkaliH₂PO₄, wobei Alkali für Lithium, Natrium, Kalium, Rubidium oder Cäsium steht, Polyphosphorsäure, C₁-C₁₂-Alkylsulfonsäure, C₆-C₁₀-Arylsulfonsäure, Trifluormethansulfonsäure, Trifluoressigsäure oder ein Copolymer aus Tetrafluorethylen und Perfluor-3,6-dioxo-4-methyl-7-octen-sulfonsäure (Nafion) eingesetzt wird.

23. Verfahren gemäß Anspruch 21, **dadurch gekennzeichnet, dass** als saurer Katalysator ein **stark saures Ionenaustauscherharz** verwendet wird.

24. Verfahren gemäß Anspruch 23, **dadurch gekennzeichnet, dass** als saurer Katalysator ein ggf. substituiertes, vorzugsweise mit Divinylbenzol vernetztes Polystyrolsulfonsäureharz verwendet wird.

25. Verfahren gemäß Anspruch 21, **dadurch gekennzeichnet, dass** ein **heterogener saurer Katalysator** aus der Gruppe (WO₃- und Cs₂O)-haltiges Aluminiumoxid, Zeolith und Montmorrillonit verwendet wird.

26. Verfahren gemäß Anspruch 21, **dadurch gekennzeichnet, dass** ein **Lewissäure-Katalysator** verwendet wird.

27. Verfahren gemäß Anspruch 26, **dadurch gekennzeichnet, dass** als Lewissäure-Katalysator mindestens eine **niedermolekulare Lewissäure** ausgewählt aus der Gruppe umfassend AlCl₃, ZnCl₂, BF₃*OEt₂, SnCl₂ und FeCl₃ verwendet wird.

28. Verfahren gemäß einem oder mehreren der Ansprüche 9 bis 27, **dadurch gekennzeichnet, dass** man die Reaktion in Schritt b) in **Lösung und/oder in Suspension** in einem organischen Lösungsmittel durchführt.

29. Verfahren gemäß Anspruch 28, **dadurch gekennzeichnet, dass** man Wasser und/oder mindestens ein niedermolekulares organisches Lösungsmittel ausgewählt aus der Gruppe bestehend aus C₃- bis C₆-Ketonen, vorzugsweise MIBK oder Aceton, geradekettige oder verzweigte C₁- bis C₄- Alkohole, C₄- bis C₁₀-Carbonsäureester, vorzugsweise Essigsäure-ethylester oder -butylester, C₃- bis C₆-Carbonsäureamide, vorzugsweise DMF oder Dimethylacetamid, C₆- bis C₁₀-Aromaten, vorzugsweise Toluol und C₃- bis C₇-cyclische Carbonate, vorzugsweise Ethylencarbonat Propylencarbonat oder Butylencarbonat, verwendet.

30. Verfahren gemäß einem oder mehreren der Ansprüche 9 bis 29, **dadurch gekennzeichnet, dass** man die Hydrolyse **in Schritt c)** in wäßriger **Lösung und/oder Suspension** durchführt.

31. Verfahren gemäß Anspruch 30, **dadurch gekennzeichnet, dass** man zusätzlich mindestens ein niedermolekulares organisches Lösungsmittel ausgewählt aus der Gruppe bestehend aus C₃- bis C₆-Ketonen, vorzugsweise MIBK oder Aceton, geradekettige oder verzweigte C₁- bis C₄-Alkohole, C₄- bis C₁₀-Carbonsäureester, vorzugsweise Essigsäure-ethylester oder -butylester, C₃- bis C₆-Carbonsäureamide, vorzugsweise DMF oder Dimethylacetamid, C₆- bis C₁₀-Aromaten, vorzugsweise Toluol und C₃- bis C₇-cyclische Carbonate, vorzugsweise Ethylencarbonat, Propylencarbonat oder Butylencarbonat, einsetzt.

32. Verfahren gemäß einem oder mehreren der Ansprüche 9 bis 31, **dadurch gekennzeichnet, dass** man die Reaktion in Schritt c) bei einer **Temperatur** von 90 bis 180 °C, vorzugsweise bei 100 bis 160 °C, insbesondere bei 120 bis 150 °C, ganz besonders bevorzugt bei 130 bis 140 °C durchführt.

33. Verfahren gemäß einem oder mehreren der Ansprüche 9 bis 32, **dadurch gekennzeichnet, dass** man die Reaktion in Schritt c) zusätzlich in Gegenwart eines sauren, basischen oder Lewissäure-Katalysators oder einer Kombination aus sauren und Lewissäure-Katalysators durchführt.

34. Verfahren gemäß einem oder mehreren der Ansprüche 1 bis 33, **dadurch gekennzeichnet, dass** das eingesetzte L-Homoserin über Fermentation hergestellt worden ist.

35. Verfahren gemäß einem oder mehreren der Ansprüche 1 bis 33, **dadurch gekennzeichnet, dass** nur Homoserin mit L-Konfiguration eingesetzt wird.

36. Verfahren nach einem oder mehreren der Ansprüche 34 oder 35 , **dadurch gekennzeichnet, dass** ein L-Homoserin haltiges, festes Produkt eingesetzt wird, das aus einer L-Homoserin haltigen Fermentationsbrühe durch Entzug von Wasser hergestellt wurde.

37. Verfahren nach Anspruch 36, **dadurch gekennzeichnet, dass** die L-Homoserin haltige Fermentationsbrühe durch Kultivierung eines L-Homoserin ausscheidenden Mikroorganismus in einem geeigneten Nährmedium hergestellt wurde.

38. Verfahren nach Anspruch 37, **dadurch gekennzeichnet, dass** es sich bei dem Mikroorganismus um ein Bakterium handelt.

39. Verfahren nach Anspruch 38, **dadurch gekennzeichnet, dass** es sich um ein Bakterium, der Gattung Corynebacterium oder Escherichia handelt.

40. Verfahren gemäß einem oder mehreren der Ansprüche 36 bis 39, **dadurch gekennzeichnet, dass** die Konzentration des L-Homoserins in der Fermentationsbrühe mindestens 1 g/l beträgt.

## Claims

1. Method for production of L-methionine, D-methionine or any desired mixture of L- and D-methionine starting from homoserine, **characterized in that** L-homoserine, D-homoserine or corresponding mixtures of L- and D-homoserine of the formula I 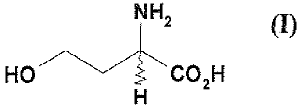 are converted to methionine by chemical transformation, without passing through any of the intermediates N-carbamoylhomoserine, 4-(2-bromoethyl)hydantoin and 4-(2-methylthioethyl)hydantoin, and
**in that** the chemical transformation of L- and/or D-homoserine is carried out in such a manner that, in a first step, by **acid-catalysed cyclization,** the corresponding 2-amino-4-butyrolactone of the formula III or salt thereof (formula IV) 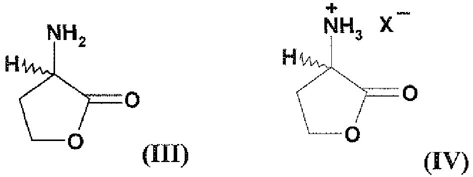 is produced, where X is Cl, Br, J, HSO₄, (SO₄)_{1/2}, H₂PO₄, (HPO₄)_{1/2}, (PO₄)_{1/3} or R'-SO₃ (where R' = methyl, ethyl, phenyl, tosyl), which is then reacted in a second step with **MeSH** to give L-methionine, D-methionine or a corresponding mixture of L- and D-methionine.

2. Method according to Claim 1, **characterized in that** use is made of an **acid catalyst** selected from the group consisting of Bronstedt acids having a pKₐ of ≤ 3.

3. Method according to Claim 2, **characterized in that**, as acid catalyst, use is made of HCl, HBr, HI, H₂SO₄, alkali metal HSO₄, H₃PO₄, alkali metal H₂PO₄, where alkali metal is lithium, sodium, potassium, rubidium or caesium, polyphosphoric acid, C₁-C₁₂-alkylsulphonic acid, C₆-C₁₀-arylsulphonic acid, trifluoromethanesulphonic acid, trifluoroacetic acid or a copolymer of tetrafluoroethylene and perfluoro-3,6-dioxo-4-methyl-7-octenesulphonic acid (Nafion).

4. Method according to Claim 1, **characterized in that**, as acid catalyst, use is made of a **strongly acidic ion-exchange resin.**

5. Method according to Claim 4, **characterized in that**, as acid catalyst, use is made of an optionally substituted, preferably by divinylbenzene, crosslinked polystyrenesulphonic acid resin.

6. Method according to Claim 1, **characterized in that** use is made of a **heterogeneous acid catalyst** from the group (WO₃- and Cs₂O)-containing aluminium oxide, zeolite and montmorrillonite.

7. Method according to Claim 1, **characterized in that** a **Lewis acid catalyst** is used.

8. Method according to Claim 7, **characterized in that**, as Lewis acid catalyst, use is made of at least one **low-molecular-weight Lewis acid** selected from the group AlCl₃, ZnCl₂, BF₃ · OEt₂, SnCl₂, FeCl₃.

9. Method for production of L-methionine, D-methionine or any desired mixture of L- and D-methionine starting from homoserine, **characterized in that** L-homoserine, D-homoserine or corresponding mixtures of L- and D-homoserine of the formula I 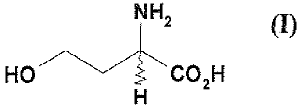 are converted to methionine by chemical transformation, without passing through any of the intermediates N-carbamoylhomoserine, 4-(2-bromoethyl)hydantoin and 4-(2-methylthioethyl)hydantoin, and **in that** the chemical transformation of L- and/or D-homoserine is carried out in such a manner that the following steps are carried out:
a) **N-acylation and cyclization** using an acylating agent to give the N-acyl-L- and/or D-homoserine lactone of the formula VIII 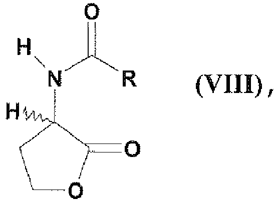 where R is hydrogen, methyl, ethyl, n-propyl, isopropyl, n-butyl, sec-butyl, tert-butyl, phenyl, mono-, di- or trihaloalkyl, where halogen = F or Cl, preferably CF₃ or CCl₃, benzyloxy or C₁- to C₄-alkyloxy, preferably tert-butyloxy, or methyloxy,
b) reaction of the N-acylhomoserine lactone obtained in step a) with MeSH in the presence of a basic or acid catalyst to give N-acylmethionine of the formula VII 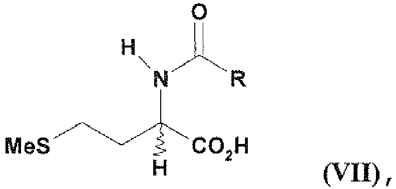
c) **hydrolysis** of the N-acylmethionine obtained in step b) to give methionine at temperatures of > 95°C.

10. Method according to Claim 9, **characterized in that**, in step a), use is made of an **acylating agent** of the general formula R-CO-X¹, where X¹ = R¹COO, OR² (R² = methyl or ethyl), Cl or Br, and R and R¹ can be identical or different and are hydrogen, methyl, ethyl, n-propyl, isopropyl, n-butyl, sec-butyl, tert-butyl, phenyl, mono-, di- or trihaloalkyl, where halogen = F or Cl, preferably CF₃ or CCl₃, benzyloxy or C₁- to C₄-alkyloxy, preferably tert-butyloxy or methyloxy.

11. Method according to Claim 9 or 10, **characterized in that**, in step a), as **solvent,** use is made of a carboxylic acid RCOOH or R¹COOH, where R or R¹ have the meaning given above, if appropriate in the presence of a further cosolvent from the group consisting of C₃ to C₆ ketones, preferably MIBK or acetone, C₄ to C₁₀ carboxylic esters, preferably ethyl or butyl acetate, C₃ to C₆ carboxamides, preferably DMF or dimethylacetamide, C₆ to C₁₀ aromatics, preferably toluene, and C₃ to C₇ cyclic carbonates, preferably ethylene carbonate, propylene carbonate or butylene carbonate.

12. Method according to one or more of Claims 9 to 11, **characterized in that**, in step a), use is made of a **pyridine derivative,** preferably dimethylaminopyridine (DMAP), or carbonyldiimidazole, as catalyst.

13. Method according to one or more of Claims 9 to 12, **characterized in that step a)** is carried out **at temperatures** of 20 to 100°C, preferably at 50 to 90°C.

14. Method according to one or more of Claims 9 to 13, **characterized in that**, in **step b),** use is made of **a basic catalyst** selected from the group consisting of tetraalkylammonium hydroxides having a maximum of 48 carbon atoms, hydroxides, carbonates, hydrogencarbonates, acetates of alkali metals or alkaline earth metals, where alkali metal is lithium, sodium, potassium, rubidium or caesium and alkaline earth metal is magnesium, calcium or barium, tertiary amines having a maximum of 36 carbon atoms and 1 to 4 nitrogen atoms, tetra(C₁-C₄-alkyl)guanidine, bicyclic amines, preferably DBU (1,8-diaza-bicyclo[5.4.0]undec-7-ene) and TBD (1,5,7-triaza-bicyclo[4.4.0]dec-5-ene), and strongly alkaline ion-exchange resins and also pyridine.

15. Method according to Claim 14, **characterized in that**, **as basic catalyst,** use is made of a trialkylamine of the general formula NR³R⁴R⁵, where R³, R⁴ and R⁵ can be identical or different and are a linear or branched C₁- to C₁₂-alkyl radical, preferably methyl, ethyl, n-propyl, isopropyl, n-butyl or sec-butyl.

16. Method according to Claim 15, **characterized in that**, as basic catalyst, use is made of N(methyl)₃, N(methyl)₂(ethyl), N (methyl) (ethyl)₂, N (ethyl)₃, N(n-propyl)₃, N(ethyl)(isopropyl)₂ or N(n-butyl)₃.

17. Method according to Claim 14, **characterized in that**, as basic catalyst, use is made of DABCO, DBU, TBD, hexamethylenetetramine, tetramethylethylenediamine or tetramethylguanidine.

18. Method according to Claim 14, **characterized in that**, as basic catalyst, use is made of R³R⁴R⁵R⁶N-hydroxide, Li-, Na-, K-, Rb-, Cs-hydroxide, Mg-, Ca-, Ba-hydroxide, where R³, R⁴, R⁵ and R⁶ can be identical or different and are a linear or branched C₁- to C₁₂-alkyl radical, preferably methyl, ethyl, n-propyl, isopropyl, n-butyl or sec-butyl.

19. Method according to Claim 14, **characterized in that**, as alkaline ion exchanger, use is made of an R⁷R⁸NR⁹-substituted crosslinked polystyrene resin, where R⁷, R⁸ and R⁹ can be identical or different and are a linear or optionally branched C₁- to C₄-alkyl radical, preferably methyl, ethyl, n-propyl, n-butyl.

20. Method according to one or more of Claims 9 to 19, **characterized in that**, in step b), use is made of 1 to 20 mol equivalents of base, calculated as hydroxide or N equivalent, preferably 1 to 10.

21. Method according to one or more of Claims 9 to 19, **characterized in that**, in **step b),** use is made of **an acid catalyst** selected from the group consisting of Brönstedt acids having a pKₐ of < 3, or Lewis acids.

22. Method according to Claim 21, **characterized in that**, as acid catalyst, use is made of HCl, HBr, HI, H₂SO₄, alkali metal HSO₄, H₃PO₄, alkali metal H₂PO₄, where alkali metal is lithium, sodium, potassium, rubidium or caesium, polyphosphoric acid, C₁-C₁₂-alkylsulphonic acid, C₆-C₁₀-arylsulphonic acid, trifluoromethanesulphonic acid, trifluoroacetic acid or a copolymer of tetrafluoroethylene and perfluoro-3,6-dioxo-4-methyl-7-octenesulphonic acid (Nafion).

23. Method according to Claim 21, **characterized in that**, as acid catalyst, use is made of a **strongly acidic ion-exchange resin.**

24. Method according to Claim 23, **characterized in that**, as acid catalyst, use is made of an optionally substituted, preferably by divinylbenzene, crosslinked polystyrenesulphonic acid resin.

25. Method according to Claim 21, **characterized in that** use is made of a **heterogeneous acid catalyst** from the group (WO₃- and Cs₂O)-containing aluminium oxide, zeolite and montmorrillonite.

26. Method according to Claim 21, **characterized in that** a **Lewis acid catalyst** is used.

27. Method according to Claim 26, **characterized in that**, as Lewis acid catalyst, use is made of at least one **low-molecular-weight Lewis acid** selected from the group comprising AlCl₃, ZnCl₂, BF₃ · OEt₂, SnCl₂ and FeCl₃.

28. Method according to one or more of Claims 9 to 27, **characterized in that** the reaction in step b) is carried out in **solution and/or in suspension** in an organic solvent.

29. Method according to Claim 28, **characterized in that** use is made of water and/or at least one low-molecular-weight organic solvent selected from the group consisting of C₃ to C₆ ketones, preferably MIBK or acetone, straight-chain or branched C₁ to C₄ alcohols, C₄ to C₁₀ carboxylic esters, preferably ethyl or butyl acetate, C₃ to C₆ carboxamides, preferably DMF or dimethylacetamide, C₆ to C₁₀ aromatics, preferably toluene, and C₃ to C₇ cyclic carbonates, preferably ethylene carbonate, propylene carbonate or butylene carbonate.

30. Method according to one or more of Claims 9 to 29, **characterized in that** the hydrolysis **in step c)** is carried out in aqueous **solution and/or suspension.**

31. Method according to Claim 30, **characterized in that** use is made additionally of at least one low-molecular-weight organic solvent selected from the group consisting of C₃ to C₆ ketones, preferably MIBK or acetone, straight-chain or branched C₁ to C₄ alcohols, C₄ to C₁₀ carboxylic esters, preferably ethyl or butyl acetate, C₃ to C₆ carboxamides, preferably DMF or dimethylacetamide, C₆ to C₁₀ aromatics, preferably toluene, and C₃ to C₇ cyclic carbonates, preferably ethylene carbonate, propylene carbonate or butylene carbonate.

32. Method according to one or more of Claims 9 to 31, **characterized in that** the reaction in step c) is carried out at a **temperature** of 90 to 180°C, preferably at 100 to 160°C, in particular at 120 to 150°C, very particularly preferably at 130 to 140°C.

33. Method according to one or more of Claims 9 to 32, **characterized in that** the reaction in step c) is carried out in addition in the presence of an acid, basic or Lewis acid catalyst, or a combination of acid and Lewis acid catalyst.

34. Method according to one or more of Claims 1 to 33, **characterized in that** the L-homoserine used has been produced via fermentation.

35. Method according to one or more of Claims 1 to 33, **characterized in that** use is only made of homoserine having L configuration.

36. Method according to one or more of Claims 34 and 35, **characterized in that** use is made of an L-homoserine-containing solid product which was produced from an L-homoserine-containing fermentation broth by removal of water.

37. Method according to Claim 36, **characterized in that** the L-homoserine-containing fermentation broth was produced by culturing an L-homoserine-excreting microorganism in a suitable nutrient medium.

38. Method according to Claim 37, **characterized in that** the microorganism is a bacterium.

39. Method according to Claim 38, **characterized in that** it is a bacterium of the genus Corynebacterium or Escherichia.

40. Method according to one or more of Claims 36 to 39, **characterized in that** the concentration of the L-homoserine in the fermentation broth is at least 1 g/l.

## Revendications

1. Procédé de fabrication de L-méthionine, de D-méthionine ou d'un mélange quelconque de L- et D-méthionine à partir d'homosérine, **caractérisé en ce que** de la L-homosérine, de la D-homosérine ou des mélanges appropriés de L- et D-homosérine de formule I 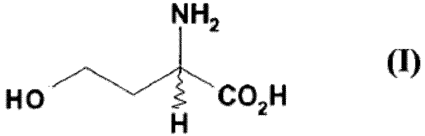 sont transformés par réaction chimique en méthionine, en ne passant par aucun des intermédiaires N-carbamoylhomosérine, 4-(2-bromoéthyl)-hydantoïne et 4-(2-méthylthioéthyl)-hydantoïne, et **en ce que** la réaction chimique de la L- et/ou D-homosérine est réalisée de sorte que, lors d'une première étape, la 2-amino-4-butyrolactone correspondante de formule III ou son sel (formule IV) soit fabriquée par cyclisation sous catalyse acide 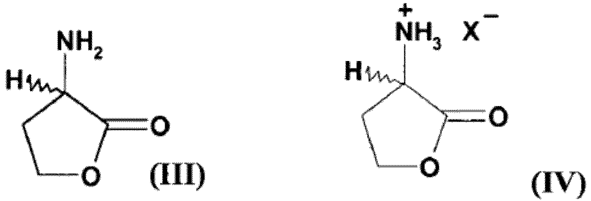 X représentant Cl, Br, I, HSO₄, (SO₄)_{1/2}, H₂PO₄, (HPO₄)_{1/2}, (PO₄)_{1/3} ou R'-SO₃ (avec R' = méthyle, éthyle, phényle, tosyle), qui est ensuite mise en réaction lors d'une seconde étape avec MeSH pour former de la L-méthionine, de la D-méthionine ou un mélange approprié de L- et D-méthionine.

2. Procédé selon la revendication 1, **caractérisé en ce qu'**un catalyseur acide choisi dans le groupe constitué par les acides de Bronsted ayant un pKa ≤ 3 est utilisé.

3. Procédé selon la revendication 2, **caractérisé en ce qu'**en tant que catalyseur acide, HCl, HBr, HI, H₂SO₄, alcali-HSO₄, H₃PO₄, alcali-H₂PO₄, alcali représentant lithium, sodium, potassium, rubidium ou césium ; l'acide polyphosphorique, un acide alkyle en C₁-C₁₂-sulfonique, un acide aryle en C₆-C₁₀-sulfonique, l'acide trifluorométhanesulfonique, l'acide trifluoroacétique ou un copolymère de tétrafluoroéthylène et d'acide perfluoro-3,6-dioxo-4-méthyl-7-octène-sulfonique (Nafion) est utilisé.

4. Procédé selon la revendication 1, **caractérisé en ce qu'**en tant que catalyseur acide, une résine échangeuse d'ions fortement acide est utilisée.

5. Procédé selon la revendication 4, **caractérisé en ce qu'**en tant que catalyseur acide, une résine d'acide polystyrène-sulfonique éventuellement substituée, de préférence réticulée avec du divinylbenzène, est utilisée.

6. Procédé selon la revendication 1, **caractérisé en ce qu'**un catalyseur acide hétérogène du groupe constitué par un oxyde d'aluminium contenant (WO₃ et C_{S2}O), une zéolithe et la montmorrillonite est utilisé.

7. Procédé selon la revendication 1, **caractérisé en ce qu'**un catalyseur acide de Lewis est utilisé.

8. Procédé selon la revendication 7, **caractérisé en ce qu'**en tant que catalyseur acide de Lewis, au moins un acide de Lewis de faible poids moléculaire choisi dans le groupe constitué par AlCl₃, ZnCl₂, BF₃*OEt₂, SnCl₂, FeCl₃ est utilisé.

9. Procédé de fabrication de L-méthionine, de D-méthionine ou d'un mélange quelconque de L- et D-méthionine à partir d'homosérine, **caractérisé en ce que** de la L-homosérine, de la D-homosérine ou des mélanges appropriés de L- et D-homosérine de formule I 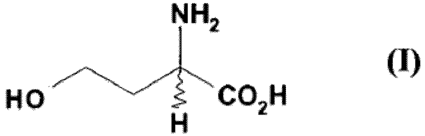 sont transformés par réaction chimique en méthionine, en ne passant par aucun des intermédiaire N-carbamoylhomosérine, 4-(2-bromoéthyl)-hydantoïne et 4-(2-méthylthioéthyl)-hydantoïne, et **en ce que** la réaction chimique de la L- et/ou D-homosérine est réalisée de sorte que les étapes suivantes soient réalisée :
a) la N-acylation et la cyclisation à l'aide d'un agent d'acylation pour former la N-acyl-L- et/ou D-homosérine-lactone de formule VIII 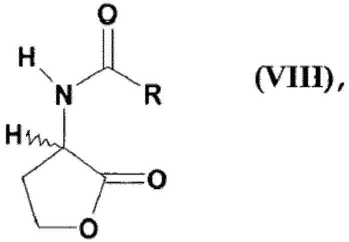 dans laquelle R signifie hydrogène, méthyle, éthyle, n-propyle, i-propyle, n-butyle, sec.-butyle, tert.-butyle, phényle, mono-, di- ou trihalogénoalkyle, avec halogène = F ou Cl, de préférence CF₃ ou CCl₃, benzyloxy ou alkyloxy en C₁ à C₄, de préférence tert.-butyloxy ou méthyloxy,
b) la mise en réaction de la N-acyl-homosérine-lactone obtenue à l'étape a) avec MeSH en présence d'un catalyseur basique ou acide pour former la N-acyl-méthionine de formule VII 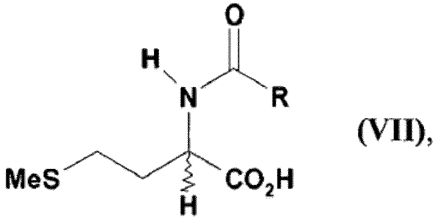
c) l'hydrolyse de la N-acyl-méthionine obtenue à l'étape b) en méthionine à des températures > 95 °C.

10. Procédé selon la revendication 9, **caractérisé en ce qu'**à l'étape a), un agent d'acylation de formule générale R-CO-X¹ est utilisé, avec X¹ = R¹COO, OR² (R² = méthyle ou éthyle), Cl ou Br, et R et R¹ pouvant être identiques ou différents et signifiant hydrogène, méthyle, éthyle, n-propyle, i-propyle, n-butyle, sec.-butyle, tert.-butyle, phényle, mono-, di- ou trihalogénoalkyle, avec halogène = F ou Cl, de préférence CF₃ ou CCl₃, benzyloxy ou alkyloxy en C₁ à C₄, de préférence tert.-butyloxy ou méthyloxy.

11. Procédé selon la revendication 9 ou 10, **caractérisé en ce qu'**à l'étape a), un acide carboxylique RCOOH ou R¹COOH est utilisé en tant que solvant, R et R¹ ayant la signification indiquée précédemment, éventuellement en présence d'un cosolvant supplémentaire du groupe constitué par les cétones en C₃ à C₆, de préférence la MIBK ou l'acétone, les esters d'acides carboxyliques en C₄ à C₁₀, de préférence l'ester éthylique ou l'ester butylique de l'acide acétique, les amides d'acides carboxyliques en C₃ à C₆, de préférence le DMF ou le diméthylacétamide,, les composés aromatiques en C₆ à C₁₀, de préférence le toluène, et les carbonates cycliques en C₃ à C₇, de préférence le carbonate d'éthylène, le carbonate de propylène ou le carbonate de butylène.

12. Procédé selon une ou plusieurs des revendications 9 à 11, **caractérisé en ce qu'**à l'étape a), un dérivé de pyridine, de préférence la diméthylaminopyridine (DMAP) ou le carbonyldiimidazole est utilisé en tant que catalyseur.

13. Procédé selon une ou plusieurs des revendications 9 à 12, **caractérisé en ce que** l'étape a) est réalisée à des températures de 20 à 100 °C, de préférence de 50 à 90 °C.

14. Procédé selon une ou plusieurs des revendications 9 à 13, **caractérisé en ce qu'** à l'étape b), un catalyseur basique choisi dans le groupe constitué par les hydroxydes de tétraalkylammonium contenant au plus 48 atomes C, les hydroxydes, carbonates, hydrogénocarbonates, acétates alcalins ou alcalino-terreux, alcalin signifiant le lithium, le sodium, le potassium, le rubidium ou le césium, et alcalino-terreux signifiant le magnésium, le calcium ou le baryum ; les amines tertiaires contenant au plus 36 atomes C et 1 à 4 atomes N, la tétra(alkyle en C₁-C₄)-guanidine, les amines bicycliques, de préférence le DBU (1,8-diazabicyclo[5.4.0]undéc-7-ène) et le TBD (1,5,7-triazabicyclo[4.4.0]déc-5-ène), et les résines échangeuses d'ions fortement alcalines, ainsi que la pyridine, est utilisé.

15. Procédé selon la revendication 14, **caractérisé en ce qu'**en tant que catalyseur basique, une trialkylamine de formule générale NR³R⁴R⁵ est utilisée, R³, R⁴ et R⁵ pouvant être identiques ou différents, et signifiant un radical alkyle en C₁ à C₁₂ linéaire ou ramifié, de préférence méthyle, éthyle, n-propyle, i-propyle, n-butyle ou sec.-butyle.

16. Procédé selon la revendication 15, **caractérisé en ce qu'**en tant que catalyseur basique, N(méthyle)₃, N(méthyle)₂(éthyle), N(méthyle)(éthyle)₂, N(éthyle)₃, N(n-propyle)₃, N(éthyle)(i-propyle)₂ ou N(n-butyle)₃ est utilisé.

17. Procédé selon la revendication 14, **caractérisé en ce qu'**en tant que catalyseur basique, DABCO, DBU, TBD, l'hexaméthylène-tétramine, la tétraméthyléthylène-diamine ou la tétraméthylguanidine est utilisé.

18. Procédé selon la revendication 14, **caractérisé en ce qu'**en tant que catalyseur basique, le N-hydroxyde de R³R⁴R⁵R⁶, l'hydroxyde de Li, Na, K, Rb, Cs, l'hydroxyde de Mg, Ca, Ba est utilisé, R³, R⁴, R⁵ et R⁶ pouvant être identiques ou différents, et signifiant un radical alkyle en C₁ à C₁₂ linéaire ou ramifié, de préférence méthyle, éthyle, n-propyle, i-propyle, n-butyle ou sec.-butyle.

19. Procédé selon la revendication 14, **caractérisé en ce qu'**en tant qu'échangeur d'ions alcalin, une résine de polystyrène réticulée substituée par R⁷R⁸NR⁹ est utilisée, R⁷, R⁸ et R⁹ pouvant être identiques ou différents, et signifiant un radical alkyle en C₁ à C₄ linéaire ou éventuellement ramifié, de préférence méthyle, éthyle, n-propyle, n-butyle.

20. Procédé selon une ou plusieurs des revendications 9 à 19, **caractérisé en ce qu'**à l'étape b), 1 à 20 équivalents molaires d'une base, calculé en tant qu'équivalent d'hydroxyde ou de N, de préférence 1 à 10, sont utilisés.

21. Procédé selon une ou plusieurs des revendications 9 à 19, **caractérisé en ce qu'** à l'étape b), un catalyseur acide choisi dans le groupe constitué par les acides de Bronsted ayant un pKa < 3 ou les acides de Lewis est utilisé.

22. Procédé selon la revendication 21, **caractérisé en ce qu'**en tant que catalyseur acide, HCl, HBr, HI, H₂SO₄, alcali-HSO₄, H₃PO₄, alcali-H₂PO₄, alcali représentant lithium, sodium, potassium, rubidium ou césium, l'acide polyphosphorique, l'acide alkyle en C₁-C₁₂-sulfonique, l'acide aryle en C₆-C₁₀-sulfonique, l'acide trifluorométhanesulfonique, l'acide trifluoroacétique ou un copolymère de tétrafluoroéthylène et d'acide perfluoro-3,6-dioxo-4-méthyl-7-octène-sulfonique (Nafion), est utilisé.

23. Procédé selon la revendication 21, **caractérisé en ce qu'**en tant que catalyseur acide, une résine échangeuse d'ions fortement acide est utilisée.

24. Procédé selon la revendication 23, **caractérisé en ce qu'**en tant que catalyseur acide, une résine d'acide polystyrène-sulfonique éventuellement substituée, de préférence réticulée avec du divinylbenzène, est utilisée.

25. Procédé selon la revendication 21, **caractérisé en ce qu'**un catalyseur acide hétérogène du groupe constitué par un oxyde d'aluminium contenant (WO₃ et C_{S2}O), une zéolithe et la montmorrillonite est utilisé.

26. Procédé selon la revendication 21, **caractérisé en ce qu'**un catalyseur acide de Lewis est utilisé.

27. Procédé selon la revendication 26, **caractérisé en ce qu'**en tant que catalyseur acide de Lewis, au moins un acide de Lewis de faible poids moléculaire choisi dans le groupe constitué par AlCl₃, ZnCl₂, BF₃*OEt₂, SnCl₂, FeCl₃ est utilisé.

28. Procédé selon une ou plusieurs des revendications 9 à 27, **caractérisé en ce que** la réaction à l'étape b) est réalisée en solution et/ou en suspension dans un solvant organique.

29. Procédé selon la revendication 28, **caractérisé en ce que** de l'eau et/ou au moins un solvant organique de faible poids moléculaire choisi dans le groupe constitué par les cétones en C₃ à C₆, de préférence la MIBK ou l'acétone, les alcools en C₁ à C₄ linéaires ou ramifiés, les esters d'acides carboxyliques en C₄ à C₁₀, de préférence l'ester éthylique ou l'ester butylique de l'acide acétique, les amides d'acides carboxyliques en C₃ à C₆, de préférence le DMF ou le diméthylacétamide, les composés aromatiques en C₆ à C₁₀, de préférence le toluène, et les carbonates cycliques en C₃ à C₇, de préférence le carbonate d'éthylène, le carbonate de propylène ou le carbonate de butylène, sont utilisés.

30. Procédé selon une ou plusieurs des revendications 9 à 29, **caractérisé en ce que** l'hydrolyse à l'étape c) est réalisée dans une solution et/ou suspension aqueuse.

31. Procédé selon la revendication 30, **caractérisé en ce qu'**au moins un solvant organique de faible poids moléculaire choisi dans le groupe constitué par les cétones en C₃ à C₆, de préférence la MIBK ou l'acétone, les alcools en C₁ à C₄ linéaires ou ramifiés, les esters d'acides carboxyliques en C₄ à C₁₀, de préférence l'ester éthylique ou l'ester butylique de l'acide acétique, les amides d'acides carboxyliques en C₃ à C₆, de préférence le DMF ou le diméthylacétmide, les composés aromatiques en C₆ à C₁₀, de préférence le toluène, et les carbonates cycliques en C₃ à C₇, de préférence le carbonate d'éthylène, le carbonate de propylène ou le carbonate de butylène, est en outre utilisé.

32. Procédé selon une ou plusieurs des revendications 9 à 31, **caractérisé en ce que** la réaction à l'étape c) est réalisée à une température de 90 à 180 °C, de préférence de 100 à 160 °C, notamment de 120 à 150 °C, de manière tout particulièrement préférée de 130 à 140 °C.

33. Procédé selon une ou plusieurs des revendications 9 à 32, **caractérisé en ce que** la réaction à l'étape c) est en outre réalisée en présence d'un catalyseur acide, basique ou acide de Lewis, ou d'une combinaison de catalyseurs acides et acides de Lewis.

34. Procédé selon une ou plusieurs des revendications 1 à 33, **caractérisé en ce que** la L-homosérine utilisée a été fabriquée par fermentation.

35. Procédé selon une ou plusieurs des revendications 1 à 33, **caractérisé en ce que** seule de l'homosérine de configuration L est utilisée.

36. Procédé selon une ou plusieurs des revendications 34 ou 35, **caractérisé en ce qu'**un produit solide contenant de la L-homosérine est utilisé, qui a été fabriqué à partir d'un bouillon de fermentation contenant de la L-homosérine par extraction d'eau.

37. Procédé selon la revendication 36, **caractérisé en ce que** le bouillon de fermentation contenant de la L-homosérine a été fabriqué par culture d'un microorganisme sécrétant de la L-homosérine dans un milieu de culture approprié.

38. Procédé selon la revendication 37, **caractérisé en ce que** le microorganisme est une bactérie.

39. Procédé selon la revendication 38, **caractérisé en ce qu'**il s'agit d'une bactérie du genre Corynebacterium ou Escherichia.

40. Procédé selon une ou plusieurs des revendications 36 à 39, **caractérisé en ce que** la concentration de la L-homosérine dans le bouillon de fermentation est d'au moins 1 g/l.
